(19) Europäisches Patentamt
European Patent Office
Office européen des brevets

(11) Publication number: **0 284 105 B1**

# (12) EUROPEAN PATENT SPECIFICATION

(45) Date of publication of patent specification: **15.11.95**

(51) Int. Cl.⁶: **A61K 38/44**

(21) Application number: **88104880.5**

(22) Date of filing: **25.03.88**

Divisional application 95106995.4 filed on 25/03/88.

## (54) Human manganese superoxide dismutase and methods of treatment.

(30) Priority: **27.03.87 US 32734**
**26.02.88 US 161117**

(43) Date of publication of application:
**28.09.88 Bulletin 88/39**

(45) Publication of the grant of the patent:
**15.11.95 Bulletin 95/46**

(84) Designated Contracting States:
**AT BE CH DE FR GB GR IT LI LU NL SE**

(56) References cited:
**EP-A- 0 138 111**
**EP-A- 0 196 056**
**EP-A- 0 213 628**
**WO-A-87/01387**
**BE-A- 905 796**
**GB-A- 2 183 658**

**Beck et al.(1987), J.Cell.Biochem., Suppl.11C, abstract no.L210**

**Pariza et al.(1991), Free Rad.Res.Comm., 15(5), 297-301.**

**SUPEROXIDE AND SUPEROXIDE DISMUTASES, 1977, Academic Press, London, A.M. MICHELSON et al. pp. 129-138; 225-330**

(73) Proprietor: **BIO-TECHNOLOGY GENERAL CORPORATION**
**375 Park Avenue, Suite 3303**
**New York**
**New York 10152 (US)**

(72) Inventor: **Hartman, Jacob R.**
**30 Kedoshay Kahir Street**
**Holon (IL)**
Inventor: **Beck, Yaffa**
**Pines Street**
**Gadera (IL)**
Inventor: **Nimrod, Abraham**
**8 Eisenberg Street**
**Rehovot (IL)**

(74) Representative: **Henkel, Feiler, Hänzel & Partner**
**Möhlstrasse 37**
**D-81675 München (DE)**

EP 0 284 105 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

Throughout this application, various publications are referenced by arabic numerals within parentheses. Full citations for these references may be found at the end of the specification immediately preceding the claims. The disclosures of these publications in their entireties are hereby incorporated by reference into this application in order to more fully describe the state of art as known to those skilled therein as of the date of the invention described and claimed herein.

Superoxide dismutase (SOD) and the phenomenon of oxygen free radicals ($O_2'$ -) was discovered in 1968 by McCord and Fridovich (1). Superoxide radicals and other highly reactive oxygen species are produced in every respiring cell as by-products of oxidative damage to a wide variety of macromolecules and cellular components (for review see 2,3). A group of metalloproteins known as superoxide dismutases catalyze the oxidation-reduction reaction $2O_2'$ - + $2H^+ \rightarrow H_2O_2 + O_2$ and thus provide a defense mechanism against oxygen toxicity.

There are several known forms of SOD containing different metals and different proteins. Metals present in SOD include iron, manganese, copper and zinc. All of the known forms of SOD catalyze the same reaction. These enzymes are found in several evolutionary groups. Superoxide dismutases containing iron are found primarily in prokaryotic cells. Superoxide dismutases containing copper and zinc has been found in virtually all eucaryotic organisms (4). Superoxide dismutases containing manganese have been found in organisms ranging from microorganisms to man.

Since every biological macromolecule can serve as a target for the damaging action of the abundant superoxide radical, interest has evolved in the therapeutic potential of SOD. The scientific literature suggests that SOD may be useful in a wide range of clinical applications. These include prevention of oncogenesis and of tumor promotion, and reduction of the cytotoxic and cardiotoxic effects of anticancer drugs (10), protection of ischemic tissues (12) and protection of spermatozoa (13). In addition, there is interest in studying the effect of SOD on the aging process (14).

The exploration of the therapeutic potential of human SOD has been limited mainly due to its limited availability.

Superoxide dismutase is also of interest because of its anti-inflammatory properties (11). Bovine-derived superoxide dismutase (orgotein) has been recognized to possess anti-inflammatory properties and is currently marketed in parts of Europe as a human pharmaceutical. It is also sold in the United States as a veterinary product, particularly for the treatment of inflamed tendons in horses. However, supplies of orgotein are limited. Prior techniques involving recovery from bovine or other animal cells have serious limitations and the orgotein so obtained may produce allergic reactions in humans because of its non-human origin.

Copper zinc superoxide dismutase (CuZn SOD) is the most studied and best characterized of the various forms of superoxide dismutase.

Human CuZn SOD is a dimeric metalloprotein composed of identical non-covalently linked subunits, each having a molecular weight of 16,000 daltons and containing one atom of copper and one of zinc (5). Each subunit is composed of 153 amino acids whose sequence has been established (6,7).

The cDNA encoding human CuZn superoxide dismutase has been cloned (8). The complete sequence of the cloned DNA has also been determined (9). Moreover, expression vectors containing DNA encoding superoxide dismutase for the production and recovery of superoxide dismutase in bacteria have been described (24,25). The expression of a superoxide dismutase DNA and the production of SOD in yeast has also been disclosed (26).

Recently, the CuZn SOD gene locus on human chromosome 21 has been characterized (27) and recent developments relating to CuZn superoxide dismutase have been summarized (28).

Much less is known about manganese superoxide dismutase (MnSOD). The manganese superoxide dismutase of E. coli K-12 has recently been cloned and mapped (22). Barra et al. disclose a 196 amino acid sequence for the manganese superoxide dismutase polypeptide isolated from human liver cells (19). Prior art disclosures differ, however, concerning the structure of the manganese superoxide dismutase molecule, particularly whether it has two or four identical polypeptide subunits (19,23). It is clear, however, that the manganese superoxide dismutase polypeptide and the CuZn SOD polypeptide are not homologous (19). The amino acid sequence homologies of MnSODs and FeSOD from various sources have also been compared (18).

Baret et al. disclose in a rat model that the half life of human manganese superoxide dismutase is substantially longer than the half-life of human copper SOD; they also disclose that in the rat model, human

manganese superoxide dismutase and rat copper SOD are not effective as anti-inflammatory agents whereas bovine copper SOD and human copper SOD are fully active (20).

McCord et al. disclose that naturally occurring human manganese superoxide dismutase protects human phagocytosing polymorphonuclear (PMN) leukocytes from superoxide free radicals better than bovine or porcine CuZn superoxide dismutase in "in vitro" tests (21).

Coassigned Belgium Patent BE-A-905,796 (March 16, 1987) discloses the native human manganese superoxide dismutase and pharmaceutical uses therefore, but does not disclose the uses claimed herein.

International Patent Application Publication No. WO87/01387 reports on the use of extracellular superoxide dismutase and Cu/Zn-superoxide dismutase for treating arthritis, but does not teach or suggest the use of human manganese superoxide dismutase for such a use as claimed herein.

The present invention concerns the preparation of a cDNA molecule encoding the human manganese superoxide dismutase polypeptide or an analog or mutant thereof.

It is also directed to inserting this cDNA into efficient bacterial expression vectors, to producing human manganese superoxide dismutase polypeptide, analog, mutant and enzyme in bacteria, to recovering the bacterially produced human manganese superoxide dismutase polypeptide, analog, mutant or enzyme. This invention is also directed to the human manganese superoxide dismutase polypeptides, analogs, or mutants thereof so recovered and their uses.

This invention further provides a method for producing enzymatically active human manganese superoxide dismutase in bacteria, as well as a method for recovering and purifying such enzymatically active human MnSOD.

The present invention also relates to a DNA molecule encoding the human manganese superoxide dismutase gene. It is also directed to inserting the DNA into mammalian cells to produce manganese superoxide dismutase polypeptide, analog, mutant and enzyme.

The present invention also relates to using human manganese superoxide dismutase or analogs or mutants thereof to catalyze the reduction of superoxide radicals to hydrogen peroxide and molecular oxygen. In particular, the present invention concerns using bacterially produced manganese superoxide dismutase or analogs or mutants thereof to reduce reperfusion injury following ischemia and prolong the survival period of excised isolated organs.

The invention further concerns the use of bacterially produced human manganese superoxide dismutase, human manganese superoxide dismutase, or human manganese superoxide dismutase mutants to treat inflammations. In particular, the invention relates to the use of human manganese superoxide dismutase, human manganese superoxide dismutase analogs or human manganese superoxide dismutase mutants to treat disorders associated with the generation of oxygen free radicals in quantities to cause undesirable symptoms. Such disorders treated include: (1) synovial inflammation induced by bacterial lipopolysaccharide endotoxin (LPS); (2) inflammation caused by adjuvant-induced arthritis; or (3) bleomycin-induced lung fibrosis. The invention further relates to methods of administering the human manganese superoxide dismutase, the human manganese superoxide dismutase analogs or human manganese superoxide dismutase mutants and to amounts and doses of human manganese superoxide dismutase, human manganese superoxide dismutase analogs or human manganese superoxide dismutase mutants.

The invention also concerns various human manganese superoxide dismutase analogs in which at least one of the human manganese superoxide dismutase polypeptides differs from naturally-occurring human manganese superoxide dismutase by the absence of one or more amino acids at the N-terminus. These analogs may be used in compositions and the compositions may be used in methods of treating various disorders by alleviating the undesirable symptoms associated with the generation of oxygen free radicals.

## SUMMARY OF THE INVENTION

Subject-matter of the present invention is the use of human manganese superoxide dismutase polypeptide or analog or mutant thereof for the production of a pharmaceutical composition for alleviating symptoms in a subject suffering from synovial inflammation, arthritis or lung fibrosis.

Veterinary and pharmaceutical compositions containing human manganese superoxide dismutase or analogs or mutants thereof and suitable carriers may be prepared. This human manganese superoxide dismutase or analogs or mutants may be used to catalyze the following reaction:

$$2O_2' - + 2H^+ \rightarrow H_2O_2 + O_2$$

and thereby reduce cell injury caused by superoxide radicals.

More particularly, these enzymes or analogs or mutants thereof may be used to reduce injury caused by reperfusion following ischemia, increase the survival time of excised isolated organs, or treat inflammations.

This invention is directed to a method of producing enzymatically active human manganese superoxide dismutase or an analog or mutant thereof in a bacterial cell. The bacterial cell contains and is capable of expressing a DNA sequence encoding the manganese superoxide dismutase or analog or mutant thereof. The method comprises maintaining the bacterial cell under suitable conditions and in a suitable production medium. The production medium is supplemented with an amount of $Mn^{++}$ so that the concentration of $Mn^{++}$ available to the cell in the medium is greater than about 2 ppm.

In a preferred embodiment of the invention the bacterial cell is an Escherichia coli cell containing a plasmid which contains a DNA sequence encoding for the human manganese superoxide dismutase polypeptide e.g. pMSE-4 or pMSΔRB4 in E. coli strain A4255. The concentration of $Mn^{++}$ in the production medium ranges from about 50 to about 1500 ppm, with concentrations of 150 and 750 ppm being preferred.

This invention also concerns a method of recovering manganese superoxide dismutase analog or mutant thereof from bacterial cells which contain the same. The cells are first treated to recover a protein fraction containing proteins present in the cells including human manganese superoxide dismutase or analog or mutant thereof and then the protein fraction is treated to recover human manganese superoxide dismutase or analog or mutant thereof. In a preferred embodiment of the invention, the cells are first treated to separate soluble proteins from insoluble proteins and cell wall debris and the soluble proteins are recovered. The soluble proteins are then treated to separate, e.g. precipitate, a fraction of the soluble proteins containing the hMnSOD or analog or mutant thereof and the fraction containing the hMnSOD or analog or mutant is recovered. The recovered fraction of soluble proteins is then treated to separately recover the human manganese superoxide dismutase or analog thereof.

A more preferred embodiment of the invention concerns a method of recovering human manganese superoxide dismutase or analog or mutant thereof from bacterial cells which contain human manganese superoxide dismutase or analog or mutant thereof. The method involves first isolating the bacterial cells from the production medium and suspending them in suitable solution having a pH of about 7.0 to 8.0. The cells are then disrupted and centrifuged and the resulting supernatant is heated for about 30 to 120 minutes at a temperature between 55 and 65 °C, preferably for 45-75 minutes at 58-62 °C and more preferably for 1 hour at 60 °C and then cooled to below 10 °C, preferably to 4 °C. Any precipitate which forms is to be removed e.g. by centrifugation, and the cooled supernatant is dialyzed against an appropriate buffer e.g. 2 mM potassium phosphate buffer having a pH of about 7.8. Preferably, the dialysis is by ultrafiltration using a filtration membrane smaller than 30K. Simultaneously with or after dialysis the cooled supernatant optionally may be concentrated to an appropriate convenient volume e.g. 0.03 of its original volume. The retentate is then eluted on an anion exchange chromatography column with an appropriate buffered solution e.g., a solution of at least 20 mM potassium phosphate buffer having a pH of about 7.8. The fractions of eluent containing superoxide dismutase are collected, pooled and dialyzed against about 40 mM potassium acetate, pH 5.5. The dialyzed pooled fractions are then eluted through a cation exchange chromatography column having a linear gradient of about 40 to about 200 mM potassium acetate and a pH of 5.5. The peak fractions containing the superoxide dismutase are collected and pooled. Optionally the pooled peak fractions may then be dialyzed against an appropriate solution e.g. water or a buffer solution of about 10 mM potassium phosphate buffer having a pH of about 7.8.

The invention also concerns purified enzymatically active human manganese superoxide dismutase or analogs thereof e.g. met-hMnSOD, or mutants produced by the methods of this invention.

The present invention also relates to a DNA molecule encoding the human manganese superoxide dismutase gene. The nucleotide sequence of the exon coding regions of one strand of the manganese superoxide dismutase gene is shown in Figure 6. The DNA encoding the manganese superoxide dismutase gene may be incorporated into a cloning vehicle such as a plasmid or a virus. The DNA or the cloning vehicle may be introduced into eucaryotic cells using known methods. The DNA encoding the human manganese superoxide dismutase gene is encoded in the plasmids pMSG11-1, pMSG4 and pMSG-1b. The eucaryotic cells which are transformed are preferably mammalian cell lines such as the human HeLa cell line or the mouse L cell line. Another aspect of this invention is the production of the human manganese superoxide dismutase polypeptide, analog, mutant or enzyme by growing the cells of this invention in suitable medium under suitable conditions.

The invention also relates to a double-stranded DNA molecule which encodes human manganese superoxide dismutase, human manganese superoxide dismutase analog and the coding strand of which has the nucleotide sequence shown in Figure 6. The invention further relates to a double-stranded molecule which encodes human manganese superoxide dismutase mutant and the coding strand of which has a

nucleotide sequence which varies from the nucleotide sequence shown in Figure 6 by one or more nucleotides.

The present invention is further concerned with eucaryotic cells in which the DNA sequence encoding human manganese superoxide dismutase, human manganese superoxide dismutase analog or human manganese superoxide dismutase mutant is expressed; and with eucaryotic cells wherein the cell has been transformed with a plasmid comprising the DNA sequence encoding human manganese superoxide dismutase, human manganese superoxide dismutase analog or human manganese superoxide dismutase mutant. This DNA sequence may be present in single or multiple copies. The DNA sequence encoding human manganese superoxide dismutase, human manganese superoxide dismutase analog or human manganese superoxide dismutase mutant is derived from a fragment of human genomic DNA containing the human manganese superoxide dismutase gene. In addition, the DNA sequence encoding human manganese superoxide dismutase is regulated by the natural regulatory elements of human genomic manganese superoxide dismutase, human manganese superoxide dismutase analog or human manganese superoxide dismutase mutant. An example of a DNA sequence coding for the human manganese superoxide dismutase, the analog or mutant and coding for the native regulatory elements regulating the gene is shown in the DNA sequence of Figure 6.

The invention is concerned with the use of human manganese superoxide dismutase, human manganese superoxide dismutase analogs or human manganese superoxide dismutase mutants for the production of a pharmaceutical composition to treat inflammation, in particular, synovial inflammation, induced by lipopolysaccharide endotoxin (LPS); inflammation caused by adjuvant-induced arthritis; and bleomycin-induced lung fibrosis. The use of manganese superoxide dismutase, manganese superoxide dismutase analogs and manganese superoxide dismutase mutants to treat these various disorders is discussed in greater detail in the Examples which follow. The invention also contemplates amounts and doses of the human manganese superoxide dismutase, analogs or mutants thereof used to alleviate symptoms and treat disorders and to methods of administration.

The invention is further concerned with human manganese superoxide dismutase analogs in which one of the human manganese superoxide dismutase polypeptides differs from naturally-occurring human manganese superoxide dismutase by the absence of one or more amino acids from the N-terminus.

Compositions comprising human manganese superoxide dismutase, human manganese superoxide dismutase analogs or human manganese superoxide dismutase mutants and a suitable carrier are also contemplated by this invention. These compositions may be used for treating any disorder for which the naturally-occurring superoxide dismutase may be used and for alleviating the undesirable symptoms associated with the generation of oxygen free radicals. These disorders and symptoms include, but are not limited to, reperfusion injury following ischemia or organ transplants, inflammations, bronchial pulmonary dysplasia, fibrosis caused by radiation treatment, inflammation caused by Crohn's disease or inflammation caused by collitis.

BRIEF DESCRIPTION OF THE FIGURES

Figure 1. The Sequence of Human Manganese Superoxide Dismutase cDNA

Figure 1 shows the nucleotide sequence of one strand of a double-stranded DNA molecule encoding the human manganese superoxide dismutase as well as the 198 amino acid sequence of human manganese superoxide dismutase corresponding to the DNA sequence. Figure 1 also shows the nucleotide sequence of one strand of a double stranded DNA molecule encoding a prepeptide to the mature human manganese superoxide dismutase consisting of twenty-four amino acids and the amino acid sequence corresponding to that DNA sequence. Also shown are the 5′ and 3′ untranslated sequences.

Figure 2. Construction of pMSE-4: Human Manganese Superoxide Dismutase Expression Plasmid

Plasmid pMS8-4, containing manganese superoxide dismutase on an EcoRI ($R_1$) insert, was digested to completion with NdeI and NarI restriction enzymes. The large fragment was isolated and ligated with a synthetic oligomer as depicted in Figure 2. The resulting plasmid, pMS8-NN contains the coding region for the mature MnSOD, preceded by an ATG initiation codon. The above plasmid was digested with EcoRI, ends were filled in with Klenow fragment of Polymerase I and further cleaved with NdeI. The small fragment harboring the manganese superoxide dismutase gene was inserted into pSODα13 which was treated with NdeI and StuI. pSODα-13 may be obtained as described in pending co-assigned U.S. Patent Application Serial No. 644,245, filed August 27, 1984 which is hereby incorporated by reference. This generated

plasmid pMSE-4 containing the manganese superoxide dismutase coding region preceded by the cII ribosomal binding site and under the control of λ $P_L$ promoter. Plasmid pMSE-4 has been deposited with the American Type Culture Collection under ATCC Accession No. 53250.

Figure 3. Effect of $Mn^{++}$ Concentration on the Activity of Superoxide Dismutase Produced in E. Coli

The chart in Figure 3 shows the correlation between the specific activity in units/mg of recombinant soluble manganese superoxide dismutase produced by E. coli strain A4255 containing plasmid pMSE-4 under both non-induction (32° C) and induction (42° C) conditions, and the concentration of $Mn^{++}$ (parts per million) in the growth medium.

Figure 4. Construction of pMSΔRB4: Human Manganese Superoxide Dismutase Expression Plasmid

$Tet^R$ expression vector, pΔRB, was generated from pSOD$\beta_1$T-11 by complete digestion with EcoRI followed by partial cleavage with BamHI restriction enzymes. pSOD$\beta_1$T-11 has been deposited with the American Type Culture Collection (ATCC) under Accession No. 53468. The digested plasmid was ligated with synthetic oligomer

```
5'- AATTCCCGGGTCTAGATCT - 3'
3'-     GGGCCCAGATCTAGACTAG - 5'
```

resulting in pΔRB containing the λ $P_L$ promoter.

The EcoRI fragment of manganese superoxide dismutase expression plasmid pMSE-4, containing cII ribosomal binding site and the complete coding sequence for the mature enzyme, was inserted into the unique EcoRI site of pΔRB. The resulting plasmid, pMSΔRB4, contains the manganese superoxide dismutase gene under control of λ $P_L$ and cII RBS and confers resistance to tetracycline.

Figure 5. Restriction Map and Organization of Human Manganese Superoxide Dismutase Gene

The thick line represents genomic DNA with the positions of the various restriction endonucleases. The black boxes numbered I - VI are the exons. The three open bars above represent the genomic clones which contain the manganese superoxide dismutase gene.

Figure 6. Nucleotide Sequence of Human Manganese Superoxide Dismutase Gene

The coding regions and adjacent nucleotides are shown; the exons (shaded areas) were identified by comparison with the cDNA clones. The initator codon (ATG), termination codon (TAA) and the polyadenylation signal (AATAAA) are underlined. The SpI hexanucleotide (GGGCGG) binding site is indicated by a line above the sequence. Dotted arrows represent possible stem-loop structures; straight arrows are direct repeats. It should be noted that the numbers shown to the left of the figure are arbitrary and were selected only to assist in the identification of the regions mentioned in the text. Neither the entire non-coding or the entire coding region is shown.

Figure 7. Exon-Intron Junctions of Human Manganese Superoxide Dismutase Gene

The nucleotide sequence at the borders of all five introns are compared with the consensus sequences. Note that a shift of one nucleotide in intron #1 may alter either the donor or acceptor sequences.

Figure 8. Pharmacokinetics of Manganese Superoxide Dismutase After Subcutaneous Injection Into Rats

Time course of the serum levels of SOD enzymatic activity in rats after subcutaneous administration of 50 mg/kg of CuZn SOD (lower curve) or manganese superoxide dismutase (upper curve). Values are expressed as the mean and standard error (3 rats per point) of the enzymatic activity, calculated as ug/ml assuming a specific activity of 3000 units/mg.

Figure 9. Comparison Between Manganese Superoxide Dismutase and CuZn SOD

Effect of CuZn SOD and manganese superoxide dismutase administration on carrageenan-induced paw swelling in rats. manganese superoxide dismutase (50 mg/kg) was administered subcutaneously 24 hours before carrageenan injection (-24 h Mn); CuZn SOD (50 mg/kg) was administered subcutaneously 2 hours (-2 h Cu) or 24 hours (-24 h Cu) prior to carrageenan injection. The control rats received carrageenan only. The bars and vertical brackets represent the means ± standard errors (8 rats per group) of the increase in paw volume 1, 2, 3 and 4 hours after carrageenan administration. The asterisks indicate the statistical significance of the difference between the treated groups compared to the control group: (*) p 0.05; (**) p 0.01; (***) p 0.001.

Figure 10. Pharmacokinetics of Recombinant Manganese Superoxide Dismutase in Rats

Pharmacokinetic behavior of recombinant manganese superoxide dismutase in rats after its administration via three routes: curve (1) depicts intravenous injection (i.v.) of 25 mg/kg body weight (b.w.); curve (2) depicts intraperitoneal (i.p.) injection of 25 mg/kg b.w.; curve (3) depicts subcutaneous (s.c.) injection of 25 mg/kg b.w.

Rats (7 rats per group) were injected at Time 0, and at Times 0.03, 0.5, 2, 4, 24, 30 and 48 hours (h) after injection blood samples were taken and analyzed both enzymatically and by RIA for the amount of manganese superoxide dismutase present in the serum of each rat. The mean value (± the standard error) for each sample is plotted against the time after injection.

Figure 11. Therapeutic Effect of Recombinant Manganese Superoxide Dismutase and CuZn Superoxide Dismutase Analog in Treating Inflammation

Bar-graph depicting the therapeutic effect of manganese superoxide dismutase and CuZn SOD on knee-joint inflammation induced by bacterial lipopolysaccharide (LPS). Knee joint inflammation is expressed as Synovial Weight (gm). Five groups of rats were used in the study: Group 1 animals (positive control; LPS) were given only LPS with no manganese superoxide dismutase treatment; Group 2 (negative control) animals were treated with saline only as sham-injection; Group 3 animals were treated with 5 mg/knee of CuZn together with the LPS; Group 4 were treated with 1 mg/knee of CuZn SOD together with LPS; Group 5 were treated with 0.6 mg/knee of manganese superoxide dismutase together with LPS. Statistical testing (Student t-test) was carried out to determine the significance of the manganese superoxide dismutase and CuZn SOD effect in inhibiting LPS-induced inflammation.

Figure 12. Effect of Recombinant Manganese Superoxide Dismutase and CuZn SOD Analog in Treating Adjuvant Induced Arthritis

Bar-graph depicting the effect of manganese superoxide dismutase and CuZn SOD on inhibiting adjuvant (Freund's adjuvant) - induced arthritis. Extent of arthritis is expressed as the increase in paw volume following adjuvant injection. The increase in paw volume is calculated relative to the paw volume 14 days after adjuvant injection; this is the period of immunologically-induced chronic arthritis. Manganese Superoxide Dismutase and CuZn SOD were administered in doses according to body weight (b.w.) to adjuvant treated rats beginning 14 days post-adjuvant injection. Seven groups of rats were used in the study (6 rats per group). All animals were injected on Day 1 with 50 $\mu$l Freund's adjuvant containing 10 mg/ml killed Mycobacterium Tuberculosis. On days 14-20, the following treatments were carried out: group 1 (control) rats received no treatment; group 2 rats received 12.5 mg/kg b.w. doses of manganese superoxide dismutase each day for 7 days; group 3 rats received 25 mg/kg b.w. of manganese superoxide dismutase each day for 7 days; group 4 rats received 12.5 mg/kg b.w. of CuZn SOD each day for 7 days; group 5 rats received 25 mg/kg b.w. of CuZn SOD each day for 7 days; group 6 rats received 50 mg/kg b.w. of manganese superoxide dismutase every second (alternate) day, that is 3 manganese superoxide dismutase treatments on days 1, 3, 5; group 7 rats received 50 mg/kg b.w. of CuZn SOD every second (alternate) day (days 1, 3, 5).

At day 21 the animals were examined for amount of paw swelling relative to day 14. Statistical analysis was carried out according to Student t-test.

Figure 13. Therapeutic Effect of Recombinant Manganese Superoxide Dismutase in Treatment of Bleomycin Induced Lung Fibrosis

Bar graph depicting the therapeutic effect of manganese superoxide dismutase on Bleomycin-induced lung fibrosis.

The degree of Bleomycin-induced lung fibrosis is expressed in two ways: (a) the ratio of lung weight to body weight; (2) the amount of hydroxyproline per lung (µg/g wet weight).

Three groups of rats were used in this study (15 rats per group): Group 1 rats (negative control) were sham-treated and received only saline. Group 2 rats were treated with 1.5 units per rat (=1.5 mg) of Bleomycin administered intra-tracheally on Day 1. Group 3 rats (positive control) were given Bleomycin (1.5 units intra-tracheally per rat) on day 1, followed by daily subcutaneous injections of manganese superoxide dismutase (50 mg/kg body weight) on days 1-7.

Three weeks after the start of the study, the surviving animals were sacrificed and their lungs were excised. The wet weights of each lung was determined and the hydroxyproline content of each lung was assayed (using standard methods as described in Example 14). The open bars represent the ratio of lung weight to body weight and the shaded bars represent the content of hydroxyproline per wet tissue weight. The brackets represent the standard error of the mean (S.E.M.).

Figure 14. Effect of Daily Subcutaneous Injection of Recombinant Human Manganese Superoxide Dismutase on Bleomycin Induced Lung Fibrosis

Three groups of rats (100-120 grams body weight) (15 rats/group) were used. One group was given bleomycin (1.5 units/rat) intratracheally on Day 1. The second group received bleomycin as in group I as well as recombinant human manganese superoxide dismutase (50 mg/µg) subcutaneously, once daily, for 7 days. (The recombinant human manganese superoxide dismutase was administered two hours before the administration of the bleomycin. A third group (negative control) was given a single injection of saline intratracheally on Day 1.

After three weeks, all surviving animals were sacrificed, their lungs were excised and the lung collagen content (expressed as hydroxy-proline) was determined. The results are shown in Figure 14.

Figure 15. Effect of Manganese Superoxide Dismutase Administration Frequency on Bleomycin-Induced Lung Fibrosis

The experimental details are essentially the same as described in Figure 14, however, the frequency of administration of manganese superoxide dismutase was changed. The recombinant human manganese superoxide dismutase was administered by a single injection subcutaneously on Day 1 or administered subcutaneously every other day (Days 1, 3, and 5). Also, the effectiveness of the recombinant CuZn SOD analog in preventing lung fibrosis was analyzed. The results are shown in Figure 15.

Figure 16. Effect of Intraperitoneal Administered Manganese Superoxide Dismutase on Bleomycin-Induced Lung Fibrosis

The experimental details are essentially the same as described in the description of Figure 14, however, the manganese superoxide dismutase or CuZn SOD analog was administered via the intraperitoneal route rather than subcutaneously. The manganese superoxide dismutase or CuZn SOD was administered as a single dose on every other day as described in the description to Figure 15. The results are shown in Figure 16.

## DETAILED DESCRIPTION OF THE INVENTION

Subject-matter of the present invention is the use of human manganese superoxide dismutase, a human manganese superoxide dismutase analog or a human manganese superoxide dismutase mutant for the production of a pharmaceutical composition for alleviating symptoms in a subject suffering from synovial inflammation, arthritis or lung fibrosis.

A double-stranded DNA molecule which includes cDNA encoding human manganese superoxide dismutase polypeptide or an analog or mutant thereof has been isolated from a human T-cell DNA library. The nucleotide sequence of a double-stranded DNA molecule which encodes human manganese superoxide dismutase polypeptide or an analog or mutant thereof has been discovered. The sequence of one

strand of DNA molecule encoding the human manganese superoxide dismutase polypeptide or analog thereof is shown in Figure 1 and includes nucleotides numbered 115 to 708 inclusive. The sequence of one strand encoding human manganese superoxide dismutase analog or mutant is substantially similar to the strand encoding the human manganese superoxide dismutase polypeptide. The nucleotide sequence of the prepeptide of human manganese superoxide dismutase is also shown in Figure 1. Nucleotides numbered 43 through 114 inclusive code for this prepeptide.

The methods of preparing the cDNA and of determining the sequence of DNA encoding the human manganese superoxide dismutase polypeptide, analog or mutant thereof are known to those skilled in the art and are described more fully hereinafter. Moreover, known synthetic methods can be employed to prepare DNA molecules containing portions of this sequence.

Conventional cloning vehicles such as plasmids, e.g., pBR322, viruses or bacteriophages, e.g., can be modified or engineered using known methods so as to produce novel cloning vehicles which contain cDNA encoding human manganese superoxide dismutase polypeptide, or analogs or mutants thereof. Similarly, such cloning vehicles can be modified or engineered so that they contain DNA molecules, one strand of which includes a segment having the sequence shown in Figure 1 for human manganese superoxide dismutase polypeptide or segments substantially similar thereto. The DNA molecule inserted may be made by various methods including enzymatic or chemical synthesis.

The resulting cloning vehicles are chemical entities which do not occur in nature and may only be created by the modern technology commonly described as recombinant DNA technology. Preferably the cloning vehicle is a plasmid, e.g. pMSE-4 or pMSΔRB4. These cloning vehicles may be introduced in cells, either prokaryotic, e.g., bacterial (Escherichia coli, B.subtilis, etc.) or eucaryotic, e.g., yeast or mammalian, using techniques known to those skilled in the art, such as transformation, transfection and the like. The cells into which the cloning vehicles are introduced will thus contain cDNA encoding human manganese superoxide dismutase polypeptide or analog or mutant thereof if the cDNA was present in the cloning vehicle or will contain DNA which includes a strand, all or a portion of which has the sequence for human manganese superoxide dismutase polypeptide shown in Figure 1 or sequence substantially similar thereto if such DNA was present in the cloning vehicle.

Escherichia coli are preferred host cells for the cloning vehicles described in this invention. A presently preferred auxotrophic strain of E. coli is A1645 which has been deposited with the American Type Culture Collection in Rockville, Maryland, U.S.A. containing plasmid pApoE-Ex2, under ATCC Accession No. 39787. All deposits with the American Type Culture Collection referred to in this application were made pursuant to the Budapest Treaty on the International Recognition of the Deposit of Microorganisms.

A1645 was obtained from A1637 by selection for $Gal^+$ (ability to ferment galactose) as well as loss of tetracycline resistance. It still contains elements of phage λ. Its phenotype is C600 $r^-m^+$ $gal^+$ $thr^-$ $leu^-$ $lacZ^-$ bl (λ cI857 ΔH1 ΔBam H1 $N^+$).

A1637 was obtained from C600 by inserting transposon containing tetracycline resistance gene into the galactose operon as well as elements of phage λ including those elements responsible for cI repressor synthesis. C600 is available from the American Type Culture Collection, as ATCC Accession No. 23724.

Prototrophic strains of Escherichia coli which enable high level polypeptide expression even when grown in a minimal media are even more preferred as hosts for expression of genes encoding manganese superoxide dismutase. One presently preferred prototrophic strain is A4255. Strain A4255 containing the plasmid pMSE-4 has been deposited with the American Type Culture Collection under ATCC Accession No. 53250.

The resulting cells into which DNA encoding human manganese superoxide dismutase polypeptide or analog or mutant thereof has been introduced may be treated, e.g. grown or cultured as appropriate under suitable conditions known to those skilled in the art, so that the DNA directs expression of the genetic information encoded by the DNA, e.g. directs expression of the hMnSOD polypeptide or analog or mutant thereof, and the cell expresses the human manganese superoxide dismutase polypeptide or analog or mutant thereof which may then be recovered.

As used throughout this specification, the term "superoxide dismutase" (SOD) means an enzyme or a polypeptide acting upon superoxide or oxygen-free radicals as receptors, or which catalyze the following dismutation reaction:

$$2O_2^{\prime}- + 2H^+ \rightarrow O_2 + H_2O_2$$

The term "manganese superoxide dismutase" (MnSOD) as used herein means any superoxide dismutase molecule containing the element manganese, in any of its chemical forms.

The term "human manganese superoxide dismutase polypeptide" as used herein means a polypeptide of 198 amino acids a portion of the amino acid sequence of which is shown in Figure 1; the N-terminus of the sequence is the lysine encoded by nucleotides 115-117 of Figure 1 and the COOH terminus of the sequence is the lysine encoded by nucleotides 706-708 of Figure 1.

The term "polypeptide manganese complex" as used herein means a molecule which includes a human manganese superoxide dismutase polypeptide in a complex with manganese in any of its chemical forms and which has the enzymatic activity of naturally-occurring human manganese superoxide dismutase.

The term "human manganese superoxide dismutase" as used herein means a molecule which includes at least two human manganese superoxide dismutase polypeptides in a complex with manganese in any of its chemical forms and which has the enzymatic activity of naturally-occurring human manganese superoxide dismutase.

The term "human manganese superoxide dismutase polypeptide analog" as used herein means a polypeptide which includes a human manganese superoxide dismutase polypeptide to either or both ends of which one or more additional amino acids are attached or removed.

The term "polypeptide manganese complex analog" as used herein means a molecule which includes a polypeptide manganese complex, the polypeptide portion of which includes one or more additional amino acids attached or removed from it at either or both ends.

The term "human manganese superoxide dismutase analog" as used herein means a molecule that includes at least two polypeptides at least one of which is human manganese superoxide dismutase polypeptide analog, in a complex with manganese in any of its chemical forms, and which has the enzymatic activity of naturally-occurring human manganese superoxide dismutase.

The term "human manganese superoxide dismutase polypeptide mutant" as used herein means a polypeptide having an amino acid sequence substantially identical to that of the human manganese superoxide dismutase polypeptide but differing from it by one or more amino acids.

The term "polypeptide manganese complex mutant" means a molecule which includes a human manganese superoxide dismutase polypeptide mutant in a complex with manganese in any of its chemical forms and which has the enzymatic activity of manganese superoxide dismutase.

The term "human manganese superoxide dismutase mutant" as used herein means a molecule which includes at least two polypeptides at least one of which polypeptides is a human manganese superoxide dismutase polypeptide mutant in a complex with manganese in any of its chemical forms and which has the enzymatic activity of naturally-occurring human manganese superoxide dismutase.

The mutants of human manganese superoxide dismutase polypeptide and human manganese superoxide dismutase which are included may be prepared by mutating the DNA sequence shown in Figure 1, the N-terminus of which sequence is the lysine encoded by nucleotides 115-117 and the COOH terminus of which sequence is encoded by nucleotides 706-708.

The DNA may be mutated by methods known to those of ordinary skill in the art, e.g. Bauer et al., Gene 37: 73-81 (1985). The mutated sequence may be inserted into suitable expression vectors as described herein, which are introduced into cells which are then treated so that the mutated DNA directs expression of the human manganese superoxide dismutase polypeptide mutants and the human manganese superoxide dismutase mutants.

The enzymatically active form of human manganese superoxide dismutase is believed to be a protein having at least two, and possibly four, identical subunits, each of which has approximately 198 amino acids in the sequence shown in Figure 1 for human manganese superoxide dismutase, the N-terminus of the sequence being the lysine encoded by nucleotides 115-117 of Figure 1 and the COOH terminus of the sequence being the lysine encoded by nucleotides 706-708 of Figure 1.

Human manganese superoxide dismutase or analogs or mutants thereof may be prepared from cells into which DNA or cDNA encoding human manganese superoxide dismutase, or its analogs or mutants have been introduced. This human manganese superoxide dismutase or analogs or mutants may be used to catalyze the dismutation or univalent reduction of the superoxide anion in the presence of protons to form hydrogen peroxide as shown in the following equation:

$$2O_2^{\cdot -} + 2H^+ \xrightarrow{\text{human MnSOD}} H_2O_2 + O_2$$

Veterinary and pharmaceutical compositions may also be prepared which contain effective amounts of human manganese superoxide dismutase or one or more human manganese superoxide dismutase analogs or mutant and a suitable carrier. Such carriers are well-known to those skilled in the art. The human manganese superoxide dismutase or analog or mutant thereof may be administered directly or in the form of a composition to the animal or human subject, e.g., to treat a subject afflicted by inflammations or to reduce injury to the subject by oxygen-free radicals on reperfusion following ischemia or organ transplantation. The human manganese superoxide dismutase or analog or mutant may also be added directly or in the form of a composition to the perfusion medium of an isolated organ, to reduce injury to an isolated organ by oxygen-free radicals on perfusion after excision, thus prolonging the survival period of the organ. Additionally, the human manganese superoxide dismutase or analog or mutant thereof may be used to reduce neurological injury on reperfusion following ischemia and to treat bronchial pulmonary dysplasia.

A method of producing enzymatically active human manganese superoxide dismutase or an analog or mutant thereof in a bacterial cell has also been discovered. The bacterial cell contains and is capable of expressing a DNA sequence encoding the human manganese superoxide dismutase or analog or mutant thereof. The method involves maintaining the bacterial cell under suitable conditions and in a suitable production medium. The production medium is supplemented with an amount of $Mn^{++}$ so that the concentration of $Mn^{++}$ in the medium is greater than about 2 ppm.

The bacterial cell can be any bacterium in which a DNA sequence encoding human manganese superoxide dismutase has been introduced by recombinant DNA techniques. The bacterium must be capable of expressing the DNA sequence and producing the protein product. The suitable conditions and production medium will vary according to the species and strain of bacterium.

The bacterial cell may contain the DNA sequence encoding the superoxide dismutase or analog in the body of a vector DNA molecule such as a plasmid. The vector or plasmid is constructed by recombinant DNA techniques to have the sequence encoding the SOD incorporated at a suitable position in the molecule.

In a preferred embodiment the bacterial cell is an Escherichia coli cell. A preferred auxotrophic strain of E. coli is A1645. A preferred prototrophic strain of E. coli is A4255 The E. coli cell described in this invention contains a plasmid which encodes for human manganese superoxide dismutase or an analog or mutant thereof.

In a preferred embodiment, the bacterial cell contains the plasmid pMSE-4. A method of constructing this plasmid is described in the Description of the Figures and the plasmid itself is described in Example 2. This plasmid has been deposited with the ATCC under Accession No. 53250.

In another preferred embodiment, the bacterial cell contains the plasmid pMSΔRB4. A method of constructing this plasmid is described in the Description of the Figures and the plasmid itself is described in Example 5. This plasmid may be constructed from pSOD$\beta_1$T-11 which has been deposited with the American Type Culture Collection under Accession No. 53468.

In specific embodiments, an enzymatically active human manganese superoxide dismutase analog is produced by E. coli strain A4255 cell containing the plasmid pMSE-4 and by E. coli strain A4255 cell containing the plasmid pMSΔRB4.

The suitable production medium for the bacterial cell can be any type of acceptable growth medium such as casein hydrolysate or LB (Luria Broth) medium, the latter being preferred. Suitable growth conditions will vary with the strain of E. coli and the plasmid it contains, for example E. coli A4255 containing plasmid pMSE-4 is induced at 42°C and maintained at that temperature from about 1 to about 5 hours. The suitable conditions of temperature, time, agitation and aeration for growing the inoculum and for growing the culture to a desired density before the production phase as well as for maintaining the culture in the production period may vary and are known to those of ordinary skill in the art.

The concentration of $Mn^{++}$ ion in the medium that is necessary to produce enzymatically active manganese superoxide dismutase will vary with the type of medium used.

In LB-type growth media $Mn^{++}$ concentrations of 150 ppm to 750 ppm have been found effective. It is preferred that in all complex types of growth mediums the concentration of $Mn^{++}$ in the medium is from about 50 to about 1500 ppm.

The specific ingredients of the suitable stock, culture, inoculating and production mediums may vary and are known to those of ordinary skill in the art.

Also described in this invention is a method of recovering human manganese superoxide dismutase or analog or mutant thereof from bacterial cells which contain the same. The cells are first treated to recover a protein fraction containing proteins present in the cells including human manganese superoxide dismutase or analog or mutant thereof and then the protein fraction is treated to recover human manganese superoxide dismutase or analog or mutant thereof.

In a preferred embodiment, the cells are first treated to separate soluble proteins from insoluble proteins and cell wall debris and the soluble proteins are then recovered. The soluble proteins so recovered are then treated to separate, e.g. precipitate, a fraction of the soluble proteins containing the human manganese superoxide dismutase or analog or mutant thereof and the fraction is recovered. The fraction is then treated to separately recover the human manganese superoxide dismutase or analog or mutant thereof.

The following is a description of a more preferred embodiment. First, the bacterial cells are isolated from the production medium and suspended in a suitable solution having a pH of about 7.0 or 8.0. The cells are then disrupted and centrifuged. The resulting supernatant is heated for a period of about 30 to 120 minutes at a temperature between approximately 55 to 65 °C, preferably for 45-75 minutes at 58 to 62 °C and more preferably one hour at 60 °C, and then cooled to below 10 °C, preferably to about 4 °C. Any precipitate which may form during cooling is removed, e.g. by centrifugation and then the cooled supernatant is dialysed against an appropriate buffer. Preferably the cooled supernatant is dialyzed by ultrafiltration employing a filtration membrane smaller than 30K, most preferably 10K. Appropriate buffers include 2 mM potassium phosphate buffer having a pH of about 7.8. After or simultaneously with this dialysis the cooled supernatant may optionally be concentrated to an appropriate volume, e.g. 0.03 of the supernatant's original volume has been found to be convenient. The retentate is then eluted on an anion exchange chromatography column with an appropriate buffered solution, e.g., a solution at least 20 mM potassium phosphate buffer having a pH of about 7.8. The fractions of eluent containing superoxide dismutase are collected, pooled and dialyzed against about 40 mM potassium acetate, pH 5.5. The dialysed pooled fractions are then eluted through a cation exchange chromatography column having a linear gradient of about 40 to about 200 mM potassium acetate (KOAc) and a pH of 5.5. The peak fractions containing the superoxide dismutase are collected and pooled. Optionally the pooled peak fractions may then be dialyzed against an appropriate solution, e.g. water or a buffer solution of about 10 mM potassium phosphate having a pH of about 7.8.

Also described in this invention are purified, i.e. substantially free of other substances of human origin, human manganese superoxide dismutase or analogs or mutants thereof produced by the methods described in this invention. In particular, it concerns a human manganese superoxide dismutase analog having at least two polypeptides, at least one of which polypeptides has the amino acid sequence shown in Figure 1, the N-terminus of which sequence is the lysine encoded by nucleotides 115-117 of Figure 1 and the COOH terminus of which sequence is the lysine encoded by nucleotides 706-708 of Figure 1 plus an additional methionine residue at the N-terminus (Met-hMnSOD). A preferred embodiment concerns purified Met-hMnSOD having a specific activity of 3500 units/mg.

Further, there is described the ligation of human manganese superoxide dismutase gene fragments taken from various plasmids to yield a complete human manganese superoxide dismutase gene fragment which can be introduced into mammalian cells for the production of MnSOD. The various human manganese superoxide dismutase fragments isolated from the plasmids detail the nucleotide sequence of the genomic human manganese superoxide dismutase gene including coding and adjacent nucleotides as well as a restriction map and organization of the gene.

The genomic gene commences at nucleotide 479 which is the first nucleotide of the ATG initiation codon and is underlined in Figure 6. The TAA termination codon for the genomic gene is at nucleotides 2022-2024. These numbers are arbitrary numbers merely to assist in identifying the nucleotide region.

A restriction map and organization of the genomic human manganese superoxide dismutase gene is depicted in Figure 5.

As noted, portions of genomic manganese superoxide dismutase DNA are found in each of three different clones, pMSG11-1, pMSG4, and pMSG-1b also depicted in Figure 5 and the DNA from these clones was used to map the nucleotide sequence of Figure 6. Also shown in Figures 6 and 7 are the exon and intron regions of the human manganese superoxide dismutase gene.

Also, there are described eucaryotic cells in which the DNA sequence encoding human manganese superoxide dismutase is expressed and to eucaryotic cells wherein the cell has been transformed with a plasmid comprising the DNA sequence encoding human manganese superoxide dismutase. This DNA sequence may be present in the cell in single or in multiple copies. The DNA sequence encoding human manganese superoxide dismutase is derived from a fragment of human genomic DNA containing the human manganese superoxide dismutase gene.

In addition, the DNA sequence encoding human manganese superoxide dismutase is regulated by native regulatory elements of human genomic manganese superoxide dismutase. The complex DNA sequence analog for genomic human manganese superoxdide dismutase polypeptide or an analog thereof including gene sequences coding for the native regulatory elements regulating gene expression is shown in

the non-coding region of Figure 6 in the region prior to where the prepolypeptide is encoded.

The eucaryotic cell expressing the genomic sequence of human manganese superoxide dismutase is a mammalian cell. Examples of mammalian cells which may be used to express the genomic DNA sequence are the human HeLa cell line and mouse L cells although it is to be understood that any mammalian cell line may be used.

Also, there are described methods for producing human manganese superoxide dismutase comprising growing the eucaryotic cells described above in a suitable medium under suitable conditions permitting production of the human manganese superoxide dismutase, human manganese superoxide dismutase analog, or human manganese superoxide dismutase mutant and permitting the recovery of the resulting human manganese superoxide dismutase, human manganese superoxide dismutase analog for human manganese superoxide dismutase mutant.

The subject application relates to various uses of the human manganese superoxide dismutase, human manganese superoxide dismutase analogs or human manganese superoxide dismutase mutants for the production of a pharmaceutical composition for treating a subject suffering from the following disorders.

The human superoxide dismutase, human manganese superoxide dismutase analogs or human manganese superoxide dismutase mutants may be used, for example, to treat a subject suffering from a disorder associated with the generation of oxygen free radicals in quantities sufficient to cause undesirable symptoms by administering to the subject an amount of the human manganese superoxide dismutase, analog or mutant to alleviate the undesirable symptoms. The compounds may be used to treat, for example, a subject with inflammation, and in particular, subjects having synovial inflammation. The inflammation may be induced by lipopolysaccharide endotoxin (LPS) or by any other inflammatory causing agent. The human manganese superoxide dismutase is preferably administered intraarticularly directly to the joint in amounts between about 1 to about 200 mg/joint applied in daily amounts. Other methods of administration may also be used.

Human manganese superoxide dismutase, human manganese superoxide dismutase analogs or human manganese superoxide dismutase mutants may further be used to treat arthritis and to treat the inflammation caused by arthritis. This arthritis may be induced, for example, by an adjuvant. The human manganese superoxide dismutase, the analogs or mutants are administered preferably subcutaneously in daily doses of between about 1 and about 100 mg/kg body weight of the subject and more preferably in amounts of between about 3 and about 50 mg/kg body weight of the subject. The compounds may also be administered intravenously or by the other methods known to those skilled in the art.

The human manganese superoxide dismutase, human manganese superoxide dismutase analogs or human manganese superoxide dismutase mutants may further be used to treat lung fibrosis, a symptom often associated as a side effect of bleomycin. The human manganese superoxide dismutase, analogs or mutants are preferably administered subcutaneously in daily doses of between about 1 and about 100 mg/kg body weight of the subject and more preferably in amounts of between about 3 and about 50 mg/kg body weight of the subject. The compounds may also be administered intravenously or intratracheally.

Further, in the present invention there are described specific human manganese superoxide dismutase analogs in which one of the human manganese superoxide dismutase polypeptides differs from naturally-occurring human manganese superoxide dismutase by the absence of one or more amino acids from the N-terminus.

The subject application is concerned with pharmaceutical compositions comprising the human manganese superoxide dismutase, human manganese superoxide dismutase analogs or human manganese superoxide dismutase mutants in a suitable pharmaceutical carrier.

The compositions may be used for treatment of various disorders which include, but are not limited to, treatment of reperfusion injury following ischemia or organ transplants; treatment of inflammations; treatment of arthritis; treatment of bronchial pulmonary dysplasia, treatment of inflammation caused by Crohn's disease; and treatment of inflammation caused by collitis. The compositions alleviate the undesirable symptoms associated with the generation of oxygen free radicals.

## EXAMPLES

The Examples which follow are set forth to aid in understanding the invention but are not intended to, and should not be construed to, limit its scope in any way. The Examples do not include detailed descriptions for conventional methods employed in the construction of vectors, the insertion of genes encoding polypeptides into such vectors or the introduction of the resulting plasmids into hosts. The Examples also do not include detailed description for conventional methods employed for assaying the polypeptides produced by such host vector systems or determining the identity of such polypeptides by

activity staining of isoelectric focusing (IEF) gels. Such methods are well-known to those or ordinary skill in the art and are described in numerous publications including by way of example the following:

T. Maniatis, E.F. Fritsch and J. Sombrook, Molecular Cloning: A Laboratory Manual, Cold Spring Harbor Laboratory, New York (1982).

J.M. McCord and I. Fridovich, J. Biol. Chem. 244: 6049-55 (1969).

C. Beauchamp and I. Fridovich, Anal. Biochem. 44: 276-87 (1971).

EXAMPLE 1

In order to identify manganese superoxide dismutase cDNA clones, mixed oligomer probes were synthesized according to the published amino acid sequence (18,19).

**5'-probe - 30 mer sequence from $AA_{15}$ - $AA_{24}$ (18,19)**

```
5'                             3'
TTGCATAATTTGTGCCTTAATGTGTGGTTC
      T     G     T     G
      G           G
```

**3'-probe - 32 mer sequence from $AA_{179}$-$AA_{189}$ (18)**

```
5'                                3'
TCTGTTACGTTTTCCCAGTTTATTACGTTCCA
  G  G                 G  G
```

The 5′-probe consisting of 30 nucleotides corresponds to amino acids 15 to 24 of mature MnSOD. The 3′-probe consisting of 32 nucleotides corresponds to amino acids 179 to 189 of mature manganese superoxide dismutase. The 5′-probe is a mixed probe consisting of 36 different sequences, as shown above. The 3′-probe is a mixed probe consisting of 16 different sequences as shown above. (When more than one nucleotide is shown at a given position, the DNA strand was synthesized with equimolar amounts of each of the shown nucleotides thus resulting in the mixed probe).

The 5′-probe was employed to screen 300,000 plaques of a T-cell cDNA library cloned into the λ gt-10 vector. Hybridization to phage plaque replicas immobilized on nitrocellulose filters was performed according to standard procedures (Maniatis et al. supra) except that the hybridization was performed at 50 °C in 8xSSC for 16 hrs. The filters were then washed at 50 °C with 5xSSC and 0.1% SDS. Three positive plaques were isolated and named Phi MS8, Phi MS1 and Phi MS1J.

EcoRI digests of DNA from Phi MS8 and Phi MS1 showed that they both have cDNA inserts approximately 800 bp long, which hybridize to both the 5′ and 3′ oligonucleotide probes. Phi MS1J carried only 450 bp cDNA insert which hybridized only to the 5′ end probe.

The EcoRI inserts of the three phage clones were subcloned into the EcoRI site of pBR322 thus yielding pMS8-4, pMS1-4 and pMS1J, respectively. Restriction analysis and hybridization to the 5′ and 3′ oligonucleotide probes revealed similar patterns for both pMS8-4 and pMS1-4. The following restriction map showing the 5′ → 3′ orientation has been deduced for both plasmids.

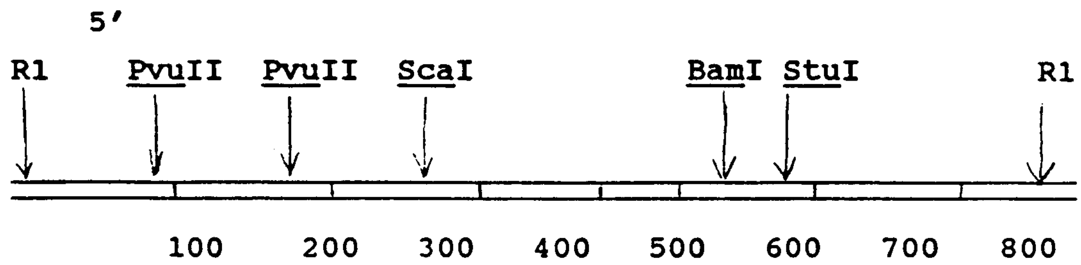


The sequence of the cDNA insert of pMS8-4 is shown in Figure 1. The predicted amino acid sequence differs from the published amino acid sequence (19) in that Glu appears instead of Gln in three (3) locations (AA 42, 88, 108) and an additional two amino acids, Gly and Trp appear between $AA_{123-124}$. Sequence analysis of pMS1-4 and pMS1J revealed that the three manganese superoxide dismutase clones were independently derived and confirmed these differences from the published amino acid sequence.

The sequence upstream of the N-terminal Lysine of mature manganese superoxide dismutase predicts a prepeptide sequence of 24 amino acids.

EXAMPLE 2

Construction of pMSE-4: $Amp^R$ Human Manganese Superoxide Dismutase Expression Plasmid

The starting point for the construction of pMSE-4 is the plasmid pMS8-4 which was obtained as described in Example 1. Plasmid pMS8-4, containing human manganese superoxide dismutase cDNA on an EcoRI insert, was digested to completion with NdeI and NarI restriction enzymes. The large fragment was isolated and ligated with a synthetic oligomer as depicted in Figure 2. The resulting plasmid, pMS8-NN contains the coding region for the mature manganese superoxide dismutase, preceded by an ATG initiation codon. The above plasmid was digested with EcoRI, ends were filled in with Klenow fragment of Polymerase I and further cleaved with NdeI. The small fragment containing the manganese superoxide dismutase gene was inserted into pSODα13 which was treated with NdeI and StuI. pSODα13 may be obtained as described in pending, co-assigned U.S. Patent Application Serial No. 644,245, filed August 27, 1984 which is hereby incorporated by reference. This generated plasmid pMSE-4 containing the manganese superoxide dismutase coding region preceded by the cII ribosomal binding site and under the control of $\lambda$ $P_L$ promoter. Plasmid pMSE-4 has been deposited with the American Type Culture Collection under ATCC Accession No. 53250. All methods utilized in the above processes are essentially the same as those described in Maniatis, supra.

EXAMPLE 3

Expression of the Recombinant Human Manganese Superoxide Dismutase

Plasmid pMSE-4 was introduced into Escherichia coli strain A4255 using known methods. Then the E. coli strain 4255, containing pMSE-4, were grown at 32°C in Luria Broth (LB) medium containing 100 µg/ml of ampicillin until the Optical Density (OD) at 600 nm was 0.7. Induction was performed at 42°C. Samples taken at various time intervals were electrophoresed separated on sodium dodecyl sulfate - polyacrylamide gels electrophoresis (SDS-PAGE). The gels showed increases in human manganese superoxide dismutase levels up to 120 minutes post-induction, at which stage the recombinant manganese superoxide dismutase protein comprised 27% of total cellular proteins as determined by scanning of Coomassie-blue stained gel. Sonication of samples for 90 sec. in a W-375 sonicator and partitioning of proteins to soluble (s) and non-soluble (p) fractions by centrifugation at 10,000 µg for 5 min. revealed that most of the recombinant manganese superoxide dismutase produced was non-soluble. The induced soluble protein fraction contained only slightly more SOD activity than the uninduced counterpart, as assayed by standard methods. See McCord et al., supra. Apparently a portion of the manganese superoxide dismutase found in the soluble fraction is inactive. This suggested that most of the human manganese superoxide dismutase produced under the conditions described in this Example is, in effect, inactive.

EXAMPLE 4

Effect of $Mn^{++}$ in Growth Media on Manganese Superoxide Dismutase Solubility and Activity

The addition of $Mn^{++}$ in increasing concentrations up to 450 ppm to the growth media of E. coli A4255, containing pMSE-4, prior to a 2 hr. induction at 42° C had no adverse effect on the overall yield of human manganese superoxide dismutase. Analysis of sonicated protein fractions soluble (s) and non-soluble (p) on sodium dodecyl sulfate polyacrylamide gel electrophoresis (SDS-PAGE), showed increased solubilization of the recombinant protein with increased $Mn^{++}$ concentrations (Table 1). An assay of superoxide dismutase activity (see McCord et al., supra) suggests a correlation between increased $Mn^{++}$ concentrations in the growth media and increased solubility of the manganese superoxide dismutase with an apparent optimum at 150 ppm $Mn^{++}$ concentration in the media (Figure 3). Furthermore increased $Mn^{++}$ concentrations activated previously inactive soluble enzyme. Soluble protein fractions of induced cultures grown at these $Mn^{++}$ levels show up to 60-fold increase in SOD activity over soluble protein fractions of non-induced cultures grown at these $Mn^{++}$ levels. Activity staining of isoelectric focusing (IEF) gels (see Beauchamp et al, supra.) revealed that multi forms of the recombinant manganese superoxide dismutase were identical to those of native human liver MnSOD.

Results for human manganese superoxide dismutase production by E. coli A1645 containing pMSE-4 were similar to those described above.

TABLE 1

| $Mn^{++}$ (ppm) | Percent Soluble human Mn SOD of Total human MnSOD Induced | Percent Soluble human Mn SOD of Soluble Bacterial Proteins | Specific Activity units/mg Soluble Proteins |
|---|---|---|---|
| 0 | 30.6 | 7.2 | 30 |
| 50 | 72.7 | 15.4 | 241 |
| 100 | 78.0 | 16.9 | 356 |
| 150 | 82.9 | 18.8 | 606 |
| 200 | 82.0 | 20.8 | 338 |
| 250 | 79.2 | 20.4 | 380 |
| 300 | 80.8 | 20.3 | 381 |
| 450 | 89.2 | 22.4 | 323 |

EXAMPLE 5

Construction of pMSΔRB4: $Tet^R$ Human Manganese Superoxide Dismutase Expression Plasmid

$Tet^R$ expression vector, pΔRB, was generated from pSODβ$_1$T-11 by complete digestion with EcoRI followed by partial cleavage with BamHI restriction enzymes. pSODβ$_1$T-11 has been deposited with the American Type Culture Collection under Accession No. 53468. The digested plasmid was ligated with synthetic oligomer

**5'- AATTCCCGGGTCTAGATCT - 3'**
**3'-     GGGCCCAGATCTAGACTAG - 5'**

resulting in pΔRB containing the λ $P_L$ promoter.

The EcoRI fragment of manganese superoxide dismutase expression plasmid pMSE-4, containing cII ribosomal binding site and the complete coding sequence for the mature enzyme, was inserted into the unique EcoRI site of pΔRB. The resulting plasmid, pMSΔRB4, contains the manganese superoxide dismutase gene under control of λ $P_L$ and cII RBS and confers resistance to tetracycline (Figure 4).

EXAMPLE 6

Expression of Human Manganese Superoxide Dismutase from pMSΔRB4

Plasmid pMSΔRB4 was introduced into Escherichia coli strain A4255, using known methods. Cultures were grown at 32°C in Luria Broth (LB) containing various concentrations of $Mn^{++}$, until the Optical Density (OD) at 600 nm reached 6.7. Induction was performed at 42°C. Samples taken at various time intervals were electrophoresed on SDS-PAGE. hMnSOD level increased with induction time up to 120 minutes, at which stage it comprised about 15% of total cellular proteins as determined by scanning of Coomassie Blue stained gel.

The induced manganese superoxide dismutase was soluble, regardless of $Mn^{++}$ concentration in growth media. This is in contrast with observations for the $Amp^R$ plasmid pMSE-4. (See Example 4.) However, maximum SOD activity and expression level were dependent on $Mn^{++}$ supplementation (Table 2).

TABLE 2

Manganese Superoxide Dismutase Expression in E.Coli A4255 (pMSΔ RB4)

| ppm $Mn^{++}$ | Percent Soluble hMnSOD of Soluble Bacterial Proteins | Specific Activity Units/mg Soluble Proteins | |
|---|---|---|---|
| | 42° | 32° | 42° |
| 0 | 10.9 | 8.0 | 23 |
| 50 | 19.8 | 8.0 | 227 |
| 100 | 16.0 | 8.0 | 241 |
| 200 | 17.0 | 10.0 | 278 |
| 300 | 16.0 | 9.3 | 238 |

EXAMPLE 7

Purification of Enzymatically Active Recombinant Human Manganese Superoxide Dismutase Analog

E. coli strain A4255 harboring plasmid pMSΔRB4 was fermented in LB supplemented with 750 ppm $Mn^{++}$, at 32°C to an A600 of 17.0. Induction of human manganese superoxide dismutase expression was effected by a temperature shift to 42°C for 2 hours at which stage the culture reached A600 of 43.0. Cells were harvested by centrifugation and resuspended in 0.2 original volume in 50 mM potassium phosphate buffer, pH 7.8 containing 250 mM NaC1. Bacteria were disrupted by a double passage through Dynomill®, centrifuged and cell debris were discarded. The supernatant was heated for 1 hour at 60°C, cooled to 4°C and the cleared supernatant was concentrated to 0.03 original volume and dialyzed against 2 mM potassium phosphate buffer, pH 7.8, on a Pellicon® ultra filtration unit equipped with a 10K membrane. The crude enzyme preparation was loaded onto a DE52® column, washed thoroughly with 2 mM potassium phosphate buffer, pH 7.8 and eluted with 20 mM potassium phosphate buffer, pH 7.8. Pooled fractions containing the enzyme were dialyzed against 40 mM potassium acetate, pH 5.5, loaded onto a CM52® column and eluted with a linear gradient of 40 - 200 mM potassium acetate, pH 5.5. Peak fractions containing human manganese superoxide dismutase were pooled, dialyzed against $H_2O$, adjusted to 10 mM potassium phosphate buffer, pH 7.8 and frozen at -20°C.

Recombinant human manganese superoxide dismutase analog obtained was more than 99% pure, with a specific activity of about 3500 units/mg. The overall yield of the purification procedure was about 30% (Table 3).

Sequencing of the purified enzyme shows the presence of an additional methionine at the N-terminal amino acid as compared with the known human manganese superoxide dismutase (19).

Analysis for metal content by atomic absorption revealed about 0.77 atoms Mn per enzyme subunit. This is in accordance with published data (23).

**TABLE 3**

**Purification of Recombinant Human* Mn-SOD**

| Step | Total Proteins gm | Yield gmSOD | % | Specific Activity units/mg |
|---|---|---|---|---|
| Dynomill® supernatant | 100.0 | 11.9 | 100.0 | 417 |
| 60°C supernatant | 24.0 | 8.2 | 68.9 | 1197 |
| Pellicon® retentate | 20.0 | 7.5 | 63.0 | 1350 |
| DE52® eluate | 7.3 | 5.7 | 48.0 | 2732 |
| CM52® eluate | 4.2 | 4.2 | 35.3 | 3500 |

*Values for enzyme purified from 15 L fermentation.

## EXAMPLE 8

### Isolation and Structure of Human Manganese Superoxide Dismutase Gene

Human placental DNA digested with HindIII and BamHI was fractionated according to its size, hybridized with an manganese superoxide dismutase cDNA probe and the positive enriched fractions were cloned in pBR322. Three distinct clones were identified according to their restriction and hybridization patterns: pMSG11-1, overlapping pMSG4, both comprising the 5′ end of manganese superoxide dismutase gene and followed by the consecutive clone pMSG-1b which contains the 3′ end of the gene. Plasmid pMSG11-1 has been deposited in the ATCC under Accession No. 67363; plasmid pMSG4 has been deposited in the ATCC under Accession No. 67364; and plasmid pMSG-1b has been deposited in the ATCC under Accession No. 67365. Figure 5 shows the restriction map and organization of the manganese superoxide dismutase gene. The nucleotide sequence of the gene is shown in Figure 6. The manganese superoxide dismutase gene spans a region of about 15 Kb and contains six exons. The first intron interrupts the region coding for the leader peptide while the last intron appears in the 3′ untranslated region downstream to the TAA termination codon.

The sequences of the donor and acceptor splice junctions at the exon-intron boundries are summarized in Figure 7 and compared with the consensus sequence. It should be pointed out that the first intron contains either an unusual donor sequence; GG instead of the highly conserved GT (as depicted in Figure 6), or an unusual acceptor sequence; GG instead of AG (if one moves the exon by one nucleotide). All other four introns are bound by the conserved GT ... AG nucleotides.

The promoter region lacks TATA and/or CAT boxes. However, it is highly rich in GC and contains eight repeats of the consensus hexanucleotide core for binding transcription factor Spl (GGGCGG). Moreover, it includes a series of direct repeats and possible stem-loop structures. The polyadenylation signal AATAAA appears 85 nucleotides downstream from the last exon (according to the known cDNA sequence). The sequence of the promoter region and the sequence of the coding regions and adjacent nucleotides was determined.

The manganese superoxide dismutase gene regions from plasmids pMSG11-1, pMSG4 and pMSG-1b can be conveniently isolated from the plasmids and ligated to one another to form the entire manganese superoxide dismutase gene. For example, the approximately 6 KB HindIII-partial BamHI fragment from pMSG11-1 can be ligated to the entire BamHI insert in plasmid pMSG4 followed by ligation to the entire

BamHI insert from pMSG-1b. The result of this ligation would be a DNA fragment encoding the human manganese superoxide dismutase gene. This DNA fragment could then be introduced into mammalian cells through known methods either directly or after ligation to a cloning vehicle such as a plasmid or virus. The transformed cell line could then be used for production of manganese superoxide dismutase polypeptide, analog or mutant thereof, by culturing in a suitable medium under suitable conditions. The polypeptide so produced could then be recovered by methods similar to those set forth in Example 7. The polypeptide so recovered could then be used formulated and used therapeutically, for example, for treatment of ischemia or inflammation.

## EXAMPLE 9

### Transcription of Manganese Superoxide Dismutase in Human Cells

The human manganese superoxide dismutase cDNA from plasmid pMS-84 (Figure 2) was hybridized to polyA$^+$ RNA from human cell lines, human placenta, mouse WEHI-3 cells and bovine liver. Two species of human mRNA for manganese superoxide dismutase were identified, a major transcript of human mRNA encoding manganese superoxide dismutase of about 1000 nucleotides (nt) long and a minor transcript of about 4000 nt in length. The mouse mRNA for manganese superoxide dismutase is similar in size to the human major transcript, whereas mRNA for bovine manganese superoxide dismutase is about 300 nt longer. The long human trancript (4000 nucleotides) hybridizes to the fifth intron of the human manganese superoxide dismutase gene, downstream from the exon coding for the carboxy terminus of the enzyme. This partially spliced transcript is non-tissue specific.

The proportion of both CuZn and manganese superoxide dismutase mRNAs in various cell lines was in the order of $10^{-3}$%, as determined by hybridization of the SOD cDNA probes to dot blots of serially diluted polyA$^+$ RNA (Table IV). The manganese superoxide dismutase message was most abundant in Hepatoma cells (2.5 x $10^{-3}$%) and CuZn SOD transcripts were most abundant in the T-lymphocyte line (4 x $10^{-3}$%).

TABLE IV

Transcription Levels of Manganese Superoxide Dismutase and CuZn SOD in Human Cell Lines

% of polyA$^+$ RNA

| | Cell Line | MnSOD | CuZnSOD |
|---|---|---|---|
| 1. | PEER T-cell | 0.6 x $10^{-3}$ | 4.0 x $10^{-3}$ |
| 2. | 5637 Bladder Carcinoma | 0.8 x $10^{-3}$ | 1.6 x $10^{-3}$ |
| 3. | Alexander Hepatoma | 2.5 x $10^{-3}$ | 2.0 x $10^{-3}$ |

EXAMPLE 10

Pharmacokinetic and Anti-Inflammatory Properties of Recombinant Human Manganese Superoxide Dismutase

INTRODUCTION

The anti-inflammatory activity of CuZn SOD has been demonstrated in various biological models. The pharmacokinetics of CuZn SOD after administration by various routes have also been examined, and it has been found to have a relatively short half-life (approximately 7 minutes when injected intravenously). By contrast, very little is known about the pharmacokinetics and biological activity of MnSOD. There is only one report on the comparison of the pharmokinetic and anti-inflammatory properties of the CuZn and the Mn containing enzymes (Baret et al., 1984). Baret et al. claim that the half-life of manganese superoxide dismutase injected intravenously is extremely long (6.45 hours). On the other hand, manganese superoxide dismutase was shown to be ineffective against carrageenan-induced paw inflammation in rats, while the CuZn SOD was effective in reducing inflammation. In the study described herein, we have compared the pharmacokinetic properties of recombinant human manganese superoxide dismutase with that of a recombinant human CuZn SOD analog when given subcutaneously. Concurrently, we have compared the anti-inflammatory activities of these two enzymes in the carrageenan paw edema model. Unexpectedly, it was found that manganese superoxide dismutase was efficacious in reducing inflammation in the rat model system.

PHARMACOKINETIC STUDIES

Rats were injected subcutaneously with 50 mg/kg body weight of either recombinant human CuZn SOD analog or recombinant human MnSOD. Blood samples were drawn 0.5, 2, 4, 8, 24, 30 and 48 hours after injection and superoxide dismutase activity in the samples was determined by an enzymatic assay (Fridovich).

Figure 8 summarizes the results. As shown, CuZn SOD analog values reached a maxmimum of about 10 µg/ml after 2 hours and stayed at about that level for additional 6 hours, but dropped to pre-injection levels after 24 hours. By contrast, manganese superoxide dismutase levels gradually increased to reach a maximal level of about 70 µg/ml by 8 hours, and stayed at about this level for at least 30 hours. By 48 hours, the enzyme activity in serum dropped to about 20 µg/ml, a value that was still well above the pre-injection levels.

ANTI-INFLAMMATORY ACTIVITY

The rat model of carrageenan-induced paw edema was used to assay the anti-inflammatory activity of CuZn SOD analog and MnSOD. In this model, Wistar-derived male rats (130-150g b.w.) were given a sub-plantar injection of 0.1 ml of 0.1% w/v carrageenan into the left hind paw. The paw volume was measured by an Hg-displacement volumeter (a modification of a Ugo-Basile® volumeter, Comerio, Italy) before and at hourly intervals after paw injection. Animals were divided into 4 groups (8 rats/group). One group received a subcutaneous injection of 50 mg/kg of manganese superoxide dismutase 24 hours before carrageenan administration (-24 h Mn; cf. Figure 9). The second group was injected with Cu/Zn SOD analog (50 mg/kg) 24 hours before the carrageenan challenge (-24 h Cu), while the third group was injected with 60 mg/Kg of CuZn SOD analog only 2 hours before the challenge (-2h Cu). The fourth group did not receive any pretreatment and served as a control.

The results are shown in Figure 9. As seen, the administration of CuZn SOD analog 2 hours before the induction of inflammation resulted in a 50% reduction of the swelling response. In contrast, pretreatment with the CuZn enzyme 24 hours before challenge was without effect. However, a 24-hour pretreatment with manganese superoxide dismutase resulted in an anti-inflammatory response which was similar to the effect of the 2 hours pretreatment with CuZn SOD analog.

CONCLUSIONS

It has been demonstrated herein that the rate of disappearance of recombinant human manganese superoxide dismutase in the rat is much lower than that of the recombinant CuZn SOD analog. This is in agreement with the previous report of Baret et al. (1984) concerning the natural human manganese

superoxide dismutase. However, the manganese-containing enzyme has been shown herein to be active in vivo as an anti-inflammatory agent - an activity that is attributed to its superoxide dismutase ability. This finding is surprising in view of the report of Baret et al. (1984), which claimed that manganese superoxide dismutase was not active in a similar system. The finding that manganese superoxide dismutase remains efficacious as an antiinflammatory drug even 24 hours after administration indicates that it may be used as a long-acting therapeutic agent.

EXAMPLE 11

Biological Model of Recombinant Manganese Superoxide Dismutase and its Role as an Anti-Inflammatory Agent: Pharmacokinetics of Intravenously Administered MnSOD

Male rats (100-110 gram body weight, group size of 7 rats) were injected intravenously with human recombinant manganese superoxide dismutase prepared in saline solution (saline = 0.15 M NaCl). Dosage of manganese superoxide dismutase was according to rat body weight (b.w.) and in this study, 25 mg/kg b.w. was the dosage of choice.

Intravenous (i.v.) administration was compared pharmacokinetically with intraperitoneal (i.p.) and subcutaneous (s.c.) administrations using the same dose (25 mg/kg b.w.) of recombinant manganese superoxide dismutase in saline and the same number of rats per group (n = 7).

After administration of manganese superoxide dismutase in the above three groups, blood samples were drawn at 0.03, 0.5, 2, 4, 24, 30 and 48 hours post-injection and were analyzed both enzymatically and by RIA. The enzyme assay was exactly as described before in Example 10 (using method of Fridovich). The RIA was carried out using standard procedures with anti-manganese superoxide dismutase antibodies (prepared by us) and protein A (from Staphylococcus aureaus).

The results are depicted in Figure 10 and may be summarized as follows:

(1) Intravenously administered (i.v., curve (1)) manganese superoxide dismutase has a very high initial serum level, as expected, of 430 μg/ml. This level drops sharply in the first two hours following injection (to 240 μg/ml). This sharp drop is due to the re-distribution (via uptake mechanisms) of manganese superoxide dismutase throughout the various tissues of the body. After the initial two hours, the rate of clearance of manganese superoxide dismutase has first-order kinetics and a half life, t1/2, of 6.2 hours.

(2) Intraperitoneally administed (i.p., curve (2)) manganese superoxide dismutase showed an initial (first 2 hours) sharp increase in serum manganese superoxide dismutase levels, consistent with rapid uptake via the peritoneal membranes, (from 0 μg/ml to 115 μg/ml). The subsequent increase in the next two hours is slow, reaching peak serum levels of 120 μg/ml after 4 hours. At this point serum manganese superoxide dismutase levels began to decline with a first-order clearance rate, and a t 1/2 of 5.5 hours.

(3) Subcutaneously administered manganese superoxide dismutase shows a low rate of entry into the serum, reaching low levels of only 27 μg/ml after 4 hours. At this point serum manganese superoxide dismutase levels decline with a first-order clearance rate and t 1/2 of 7.3 hours.

It should also be noted, especially in the case of intravenously administered recombinant manganese superoxide dismutase, that significant serum levels are still present 48 hours after injection (10 μg/ml).

Thus, we conclude that the most effective way of administering manganese superoxide dismutase is intravenously. This gives very high initial serum levels which remain as significantly high serum levels (160 μg/ml) in the first 4 hours after injection. This is much more effective than either intraperitoneally or subcutaneously-administered manganese superoxide dismutase using same dosages in injection.

Finally, in all cases, the t 1/2 of serum manganese superoxide dismutase is markedly greater than that described for CuZn SOD (about 7 - 30 minutes), thus making manganese superoxide dismutase a potentially more effective therapeutic agent than CuZn SOD.

Human manganese superoxide dismutase was reported to have a much longer half-life after intravenous administration to rats than the phylogenetically unrelated enzyme CuZn SOD (t1/2 = 6.45 h vs. 6 min, respectively; Baret, Jadot and Michelson, Biochem. Pharmacol. 33: 2755 (1984).) It was further claimed that, unlike CuZn SOD, manganese superoxide dismutase is pharmacologically inactive when tested in inflammation models in vivo. As described above, we have cloned and expressed human manganese superoxide dismutase in E. coli. The purified recombinant protein is different from the natural enzyme by the presence of an additional methionine at the amino terminus. However, it exhibits specific enzymatic activity indistinguishable from that of natural mitochrondrial manganese superoxide dismutase purified from human liver.

Pharmacokinetic analysis of recombinant human manganese superoxide dismutase (r-hMnSOD) given intravenously to rats revealed a half-life of 6.2 h, which is consistent with the published value for the natural

enzyme. The potential anti-inflammatory activity of r-hMnSOD was tested in the model of carrageenan-induced edema in the rat paw. Recombinant human CuZn SOD (r-hCuZnSOD) was used for comparison. The two enzymes were administered according to two experimental regimens: (1) the enzymes were injected subcutaneously (50 mg/kg B.W.) either 2 h or 24 h before the carrageenan challenge; (2) the enzymes were injected intravenously at 8 or 20 mg/kg B.W. 10 min. prior to the carrageenan challenge. The results are illustrated in Table 5 below:

**TABLE 5**

| Route of Injection | Time Before Carrageenan | Dose (Mg/Kg B.W.) | Inhibition of Paw Swelling | |
|---|---|---|---|---|
| | | | r-hMnSOD | r-hCuZnSOD |
| Subcutaneous | 2 h | 50 | 50% | 50% |
| Subcutaneous | 24 h | 50 | 40% | 0 |
| Intravenous | 10 min | 8 | 50% | 0 |
| Intravenous | 10 min | 20 | 80% | 10% |

Collectively, the results demonstrate that r-hMnSOD is an effective ani-inflammatory agent. Furthermore, its extended half-life in the blood, relative to CuZn SOD, makes r-hMnSOD more suitable for treatment of chronic inflammatory diseases, such as arthritis, colitis and radiation damage.

EXAMPLE 12

Biological Model of Recombinant Manganese Superoxide Dismutase and Its Role as an Anti-Inflammatory Agent: Effect of Intra-Articular Administration of Manganese Superoxide Dismutase on Synovial Inflammation Induced by Bacterial Lipopolysaccharide Endotoxin (LPS)

As a model for synovial inflammation and the ability of manganese superoxide dismutase to inhibit such inflammation, the knee-joint (synovial) inflammation model in rats was utilized. (More details used herein as reference, are described by Ginsburg et al., in "Bayer-Symposium VI: Experimental Models of Chronic Inflammatory Diseases" 256-299 (1977), Springer Verlag).

In this model, inflammation is induced by intraarticular injection of bacterial lipopolysaccharide endotoxin (LPS) (0.1 mg/rat). The degree of inflammation is reflected by the swelling of the synovial tissue, and is measured as the increase in weight 8-16 hours postadministration. Figure 11 shows a comparison between human CuZn SOD and manganese superoxide dismutase administered together with the LPS. To test the anti-inflammatory effects of manganese superoxide dismutase and CuZn SOD in this model, it is best to coadminister the LPS toxin and the manganese superoxide dismutase or CuZn SOD. Dosages that were used are as follows: 0.6 mg/knee manganese superoxide dismutase and both 1 mg/knee and 5 mg/knee for CuZn SOD. As shown, manganese superoxide dismutase was as effective as CuZn SOD in the inhibition of LPS-induced synovial inflammation. Both doses of CuZn SOD (1 mg/knee, 5 mg/knee) and the dose of manganese superoxide dismutase (0.6 mg/knee) were effective in reducing, significantly, the amount of LPS-induced knee-joint inflammation. In fact, the level of inflammation resulting from co-injection of SOD (CuZn SOD or MnSOD) and LPS is no greater than the sham-injection with saline only (negative control). This therefore, clearly illustrates the anti-inflammatory effect of SOD (MnSOD and CuZn SOD). The inhibition of inflammation was judged to be significant on the basis of Student t-test statistical analysis at 3 levels of probabilistic stringency; 5%, 1% and 0.1% levels (p 0.05; - 0.01, and p 0.001, respectively). Thus, the higher CuZn SOD dose (5 mg/knee) was most significant in its effect; the manganese superoxide

dismutase dose (0.6 mg/knee) and lower CuZn SOD dose (1 mg/knee) were highly significant in inflammation-inhibition.

EXAMPLE 13

Biological Model of Recombinant Manganese Superoxide Dismutase and Its Role as an Anti-Inflammatory Agent: Effect of Manganese Superoxide Dismutase in Adjuvant-Induced Arthritis

The induction of joint inflammation in rats by administration of Freund's adjuvant is considered to be the model of choice for experimental rheumatoid arthritis (more details, used herein as reference are described in Newbould, Brit. J. Pharmacol. 21 (1963) 127-). An injection of the adjuvant (50 µl of Freunds' adjuvant with 10 mg/ml of killed Mycobacterium Tuberculosis) into the foot pad results in an initial swelling of the paw, reaching a plateau after 3 days, followed after 14 days by a second increase in paw and joint swelling, which persists for another 7-10 days. The second period is regarded as the phase of immunologically-induced chronic arthritis. To examine the efficacy of SOD as an anti-arthritic drug, CuZn SOD and manganese superoxide dismutase were given subcutaneously (in doses expressed as mg per kg body weight) to adjuvant-treated rats during days 14-21 after adjuvant administration. The enzymes were given either daily (both 12.5 mg/kg and 25 mg/kg doses) or on alternating days (a 50 mg/kg dose). The results shown in Figure 12 indicate that manganese superoxide dismutase is more effective as the treatment given daily, while the CuZn SOD given on alternating days appeared to be ineffective. The levels of effectiveness of treatment were determined by statistical analysis using the Student t-test at 3 levels of stringency (described in Example 12). These results clearly demonstrate the efficacy of manganese superoxide dismutase as an anti-arthritic drug.

EXAMPLE 14

Biological Model of Recombinant Manganese Superoxide Dismutase and Its Role as an Anti-Inflammatory Agent: Effect of Manganese Superoxide Dismutase on Bleomycin-induced Lung Fibrosis

Bleomycin (BLM) is an antineoplastic agent widely used against various types of carcinomas and lymphomas (more details, used herein as reference, are described in Counts et al., J. Pharmacol. Exp. Ther. 219: 675-678 (1981). A major problem associated with the use of bleomycin is the induction of lung fibrosis resulting from high doses of the agent, thus limiting its therapeutic usefulness. (See, for example, Crooks & Bradner, J. Med., 7: 333 (1976)). This side-effect of bleomycin is believed to be mediated by the generation of oxygen radicals in the lung tissue. (See, for example, Frank, Trends Pharmacol. Sci. 4: 124 (1983)). To examine the possible use of manganese superoxide dismutase for the protection of the lung from bleomycin-induced fibrosis, we have used the rat as a biological model. (See, for example, Kelley et al., J. Lab. Clin. Med. 96: 254 (1980)). In this model, 15 rats (100-120 g b.w.) were treated with 1.5 mg (= 1.5 units) of bleomycin given intra-tracheally. A second group of rats (15 rats), the negative control, received saline only, while a third group (15 rats) was given bleomycin as in group 1 and then treated for 7 days with daily subcutaneous injections of manganese superoxide dismutase (50 mg/kg b.w. dose). Table 6 represents the 3 week mortality data following bleomycin administration. As seen in the table, the daily treatment with manganese superoxide dismutase was significantly protective (87% survival versus 40%; statistical significance of $p>0.05$ by Student t-test). The lungs of the surviving rats were excised and the collagen content (expressed as hydroxy-proline content) was determined, (using standard procedures according to Woessner in "The Methodology of Connective Tissue Re-search", Hall, Ed. Oxford (1976) Joynson-Bruvvers Ltd. pp. 227-233 and Woessner, Arch. Biochem. Biophys. 93: 440-447 (1961). FIGURE 13 shows the lung weights (normalized for 100 g body weight) and hydroxyproline content of the treated animals. As shown in Figure 13, the MnSOD treatment partially inhibited the increase in total lung weight and effectively protected the lungs against an increase in collagen (reflected by level of hydroxyproline).

As seen in Figures 13 and 14, OH-proline levels in the r-human manganese superoxide dismutase treated group were elevated to only 25% of the BLM group, indicating that the enzyme effectively inhibited fibrosis.

In two additional experiments, we compared the effects of frequency as well as different routes of administration using both r-human manganese superoxide dismutase and r-human CuZn SOD. In the first experiment, the enzymes were given subcutaneously (50 mg/kg) either once every other day (Days 1, 3, 5) or by a single injection on Day 1. As seen in Figure 15, r-human manganese superoxide dismutase was partially protective when given once (approx. 34% reduction of BLM result) but was more effective when

administered every other day or daily (Figures 14 and 15) for a week (nearly 75% reduction of BLM insult). Similarly, a single dose of r-human CuZn SOD showed no protection, but when given every other day was equally as protective as a single injection of r-human manganese superoxide dismutase.

The same experiment was repeated using an intraperitoneal route of administration rather than subcutaneous. The results, depicted in Figure 16, again show that the preferred combination is r-human manganese superoxide dismutase given three times a week.

Collectively, our results confirm the hypothesis that BLM in lung fibrosis is oxygen free radical mediated, and that such fibrosis could be inhibited by either r-human manganese superoxide dismutase or r-human CuZn SOD. The fact that r-hMnSOD was more effective than r-CuZnSOD in this process is most probably due to its longer half-life in circulation following intravenous injection (t 1/2 i.v. for r-hMnSOD and CuZn SOD is about 6 hours versus 6 minutes, respectively, in rats). Figure 10 represents the results of our pharmacokinetic study of r-human manganese superoxide dismutase in rats (25 mg/kg), using 3 routes of administration.

The first-order kinetic decline from peak levels showed half-lives of 6.2, 5.5 and 7.3 hours for intravenous, intraperitoneal and subcutaneous route respectively. The much shorter half-life of r-human CuZn SOD (Figure 8) requires more frequent administration and possibly higher doses as compared to r-human manganese superoxide dismutase, in order to attain equivalent pharmacological levels and hence similar protective effects. Further experiments assessing dose-response are in progress.

We conclude that the recombinant SOD enzymes, particularly r-human manganese superoxide dismutase, might be a useful adjunct to bleomycin (and possibly its derivatives, such as tallysomycin) therapy.

CONCLUSIONS

It has been demonstrated herein that recombinant manganese superoxide dismutase is an effective anti-inflammatory agent. This was shown by its therapeutic effects in three biological models of inflammation. In all three models, the recombinant manganese superoxide dismutase had a highly significant effect in inhibiting inflammation and was at least as effective (in most cases more effective) than CuZn SOD which was used for comparative purposes. In addition, the fact that manganese superoxide dismutase remains in the serum for long periods, as shown by the pharmacokinetic studies, indicates that it may be used as a long-acting therapeutic agent.

TABLE 6

Effect of MnSOD on Bleomycin-induced Death After 3 Weeks

| Treatment | No. of Surviving Rats |
|---|---|
| Bleomycin(*) | 6/15 (40%) |
| Bleomycin(*) + MnSOD(**) | 13/15 (87%)<br>($p<0.05$; Student t-test) |
| Saline (0.15 M NaCl) only | 15/15 (100%) |

* 1.5 mg (1.5 units) Bleomycin was given intra-tracheally per rat.

** 50 mg/kg MnSOD was given daily per rat (by subcutaneous injection) for 7 days on the first week after bleomycin administration.

EXAMPLE 15

Preparation of Human Manganese Superoxide Dismutase Analogs

Human manganese superoxide dismutase was produced essentially as described in Example 7. The manganese superoxide dismutase has the following amino terminal sequence.

Met-Lys-His-Ser-Leu-Pro---

As described below, the manganese superoxide dismutase (MnSOD) was treated with *Aeromonas* aminopeptidase. *Aeromonas* aminopeptidase was prepared from the extracellular filtrate of *Aeromonas proteolytica* obtained from the American Type Culture Collection (ATCC Accession No. 15338), essentially according to J.M. Prescott and S.H. Wilkes, Methods Enzymol. 46: 530-543 (1976). The purification procedure included the following steps: sedimentation and filtration of bacteria, ammonium sulfate precipitation of the filtrate (367 g per liter), acetone fractionation (43.7% to 70% acetone), heat treatment at 70 °C (for 8 hours) to destroy endopeptidase activity, gel filtration on Sephadex G-75® and ion-exchange chromatography on DEAE-Sephadex A-50®. In all experiments 10 mM Tris-Hcl buffer, pH 8.0 replaced 10 mM tricine buffer, pH 8.0 employed by Prescott and Wilkes. Pre-equilibration and elution from the Sephadex G-75® column were performed using a solution containing 5 micromolar (μM) $ZnCl_2$ rather than 50 micromolar (μM) $ZnCl_2$ employed in the original procedure (Prescott and Wilkes, *supra*). Purification on the DEAE-Sephadex A-50® column was performed by preequilibration of the column with 0.1 M NaCl in 10 mM Tris-Hcl, pH 8.0 containing 5 micromolar (μM) $ZnCl_2$, application of the sample and gradient elution with 0.6 M NaCl in the same buffer (containing 5 micromolar (μM) $ZnCl_2$). When the salt concentration had reached about 0.5M the column was eluted with 0.7M NaCl in the same buffer. The major peak which

eluted from the column was collected and dialyzed against 10 mM Tris-Hcl, 0.1M NaCl, pH 8.0 containing 5 micromolar (μM) $ZnCl_2$ and then kept frozen at -20 °C.

Prior to reaction with the human manganese superoxide dismutase (hMnSOD), the analog or mutant (of hMnSOD), the amino peptidase enzyme solution was incubated at 70° for 2 hours to inactivate any possible traces of endopeptidase activity that might have been retained in the preparation and reactivated after prolonged storage.

Amino acid analysis was performed using the method of precolumn derivatization with FMOC (9-fluoroenylmethyloxycarbonyl chloride), (method according to Josefsson and Einarsson, see, for example, Einarsson et al., J. Chromatography 282: 609-618 (1983) and Einarsson, J. Chromatography 348: 213-220 (1985)). The amino acid derivatives were separated on a reverse phase Lichrocart Supersphere® (C18) column (Merck®) in a HPLC (high pressure liquid chromatography) system (Hewlett Packard® 1090 HPLC system, operated according to the manufacturer's specification).

Depending on the pH of the solution during treatment, and depending on the duration of the treatment, one or more amino acids were removed from the N-terminal of MnSOD. The pH was varied from pH 4.5 - pH 10 and the duration of treatment was extended to as long as 24 hours. The experiment below describes the currently preferred conditions for amino acid removal from the N-terminal of MnSOD.

Met-MnSOD, 30 mg/ml, produced as described in Example 3, was dialyzed overnight in 5 mM potassium acetate (KOAc) pH 4.5.

250 microliters of Met-MnSOD solution, pH 4.5 (final concentration 6 mg/ml), 700 microliters of 5 mM KOAc buffer pH 4.5 and 50 microliters of aminopeptidase solution (1 mg/ml) were mixed and incubated at 37 °C. Two aliquots of 108 microliters were taken after 0 h, 5 h and 24 h.

To the first aliquot, 12 microliters of 15% sulfosalicylic acid solution in water were added, mixed and incubated at 37 °C for 15 min., and then centrifuged in an Eppendorf® bench centrifuge. 100 microliter sample was removed for direct amino acid analysis (without acid hydrolysis). The second aliquot was removed for N-terminal sequence analysis.

Control experiments were run in parallel by incubating Met-MnSOD in the absence of aminopeptidase as described in the above procedure. Two aliquots of 108 microliters were removed at each time point, and were treated and analyzed as described for the actual samples.

After 24 hours of incubation at 37 °C, the following molecules were obtained as illustrated in Table 7:

## TABLE 7

| Percent of Total Molecules | N-Terminal Sequence of SOD Analog |
|---|---|
| 50% | Met-Lys-His-Ser-Leu-Pro |
| 25% | Lys-His-Ser-Leu-Pro |
| 12% | His-Ser-Leu-Pro |
| Less than 10% | Ser-Leu-Pro |
| Less than 10% | Leu-Pro |

The different analogs can be used for therapeutic purposes as described in Examples 10-14.

If desired the different analogs can be purified from one another using standard techniques well-known to those skilled in the art.

## REFERENCES

1. McCord, J.M. and Fridovich, I., J. Biol. Chem. 244: 6049-55 (1969).

2. Fridovich, I. in Advances in Inorganic Biochemistry, eds. Eichhorn, G.L. and Marzilli, L.G. (Elsevier/North Holland, New York), pp. 67-90 (1979).

3. Freeman, B.A. and Crapo, J.D., Laboratory Investion 47: 412-26 (1982).
4. Steinman, H.M. in Superoxide Dismutase, ed. Oberley, L.W. (CRC Press, Florida), pp. 11-68 (1982).
5. Hartz, J.W. and Deutsch, H.F., J. Biol. Chem. 247: 7043-50 (1972).
6. Jabusch, J.R., Farb, D.L., Kerschensteiner, D.A. and Deutsch, H.F., Biochemistry 19: 2310-16 (1980).
7. Barra, D., Martini, F., Bannister, J.V., Schinina, M.W., Rotilio, W.H., Bannister, W.H. and Bossa, F., FEBS Letters 120: 53-56 (1980).
8. Lieman-Hurwitz, J., Dafni, N., Lavie, V. and Groner, Y., Proc. Natl. Acad. Sci. USA 79: 2808-11 (1982).
9. Sherman, L., Dafni, N., Lieman-Hurwitz, J. and Groner, Y., Proc. Natl. Acad. Sci. USA 80: 5465-69 (1983).
10. Oberley, L.W. and Buettner, G.R., Cancer Research 39: 1141-49 (1979).
11. Huber, W. and Menander-Huber, K.B., Clinics in Rheum. Dis. 6: 465-98 (1980).
12. McCord, J.M. and Roy, R.S., Can. J. Physiol. Pharma. 60: 1346-52 (1982).
13. Alvarez, J.G. and Storey, B.T., Biol. Reprod. 28: 1129-36 (1983).
14. Talmasoff, J.M., Ono, T. and Cutler, R.G., Proc. Natl. Acad. Sci. USA 77: 2777-81 (1980).
15. Lowry, O.H., Rosebrough, N.J., Farr, A.L. and Randall, R.J., J. Biol. Chem. 193: 265-75 (1951).
16. Weser, U. and Hartmann, H.J., FEBS Letters 17: 78-80 (1971).
17. Jewett, S.LO., Latrenta, G.S. and Beck, C.M., Arc. Biochem. Biophys. 215: 116-128 (1982).
18. Harris, J.I. and Steinman, H.M., Superoxide and Superoxide Dismutase Michelson, A.M., McCord, J.M. and Fridovich, I. eds., Academic Press, London, pp. 225-230 (1977).
19. Barra, D., Schinina, M.E., Simmaco, M., Bannister, J.V., Bannister, W.H., Rotilio, G. and Bossa, F., J. Biol. Chem. 259: 12595-601 (October 25, 1984).
20. Baret, A., Jadot, G., and Michelson, A.M., Biochemical Pharmacology 33: 2755-60 (September 1, 1984).
21. McCord, J.M. and Salin, M.L., Movement, Metabolism and Bactericidal Mechanisms of Phagocytes, Ross, A., Patriarca, P.L., Romeo, D. (eds) pp. 257-264 (1977).
22. Touati, D., Journal of Bacteriology 155: 1078-87 (1983).
23. McCord, J.M., Boyle, J.A., Day, Jr., E.D., Rizzolo, L.J. and Salin, M.L., Superoxide and Superoxide Dismutase, Michaelson, A.M., McCord, J.M., and Fridovich, I. (eds) Academic Press, London pp. 129-138 (1977).
24. European Patent Publication No. 0131843 A1, published January 23, 1985, corresponding to European Patent Application No. 84107717.5, filed July 3, 1984, which claims priority of U.S. Serial No. 514,188, filed July 15, 1983.
25. Hallewell, et al., Nucleic Acids Res. 5: (1985).
26. European Patent Publication 0138111 A1, published April 24, 1985, corresponding to European Patent Application No. 84111416.8, filed September 25, 1984, which claims priority of U.S. Serial No. 538,607, filed October 3, 1983, and U.S. Serial No. 609,412, filed May 11, 1984.
27. EMBO Journal 4(1): 77-84 (January 1985).
28. Abstracts of the Fourth International Conference on Superoxide and Superoxide Dismutase, Rome, Italy, September 1-6, 1985.

## Claims

**1.** The use of human manganese superoxide dismutase, a human manganese superoxide dismutase analog or a human manganese superoxide dismutase mutant for the production of a pharmaceutical composition for alleviating symptoms in a subject suffering from synovial inflammation, arthritis or lung fibrosis.

**2.** The use of claim 1 wherein the subject is suffering from synovial inflammation and wherein the composition is suitable for intraarticular administration.

**3.** The use of claim 2 wherein the amount of human manganese superoxide dismutase, human manganese superoxide dismutase analog, or human manganese superoxide dismutase mutant in the composition is between about 1 and about 200 mg/joint.

**4.** The use of claim 1 wherein the subject is suffering from arthritis and the symptoms comprise inflammation.

**5.** The use of claim 4 wherein the composition is suitable for daily administration.

6. The use of claim 5 wherein the amount of human manganese superoxide dismutase, human manganese superoxide dismutase analog, or human manganese superoxide dismutase mutant in the composition is between about 1 and about 100 mg/kg body weight of the subject.

7. The use of claim 6 wherein the amount of human manganese superoxide dismutase, human manganese superoxide dismutase analog, or human manganese superoxide dismutase mutant in the composition is between about 3 and about 50 mg/kg body weight of the subject.

8. The use of claim 4 wherein the composition is suitable for subcutaneous administration.

9. The use of claim 4 wherein the composition is suitable for intravenous administration.

10. The use of claim 1 wherein the subject is suffering from lung fibrosis.

11. The use of claim 10 wherein the composition is suitable for daily administration.

12. The use of claim 11 wherein the amount of human manganese superoxide dismutase, human manganese superoxide dismutase analog, or human manganese superoxide dismutase mutant in the composition is between about 1 and about 100 mg/kg body weight of the subject.

13. The use of claim 12 wherein the amount of human manganese superoxide dismutase, human manganese superoxide dismutase analog, or human manganese superoxide dismutase mutant in the composition is between about 3 and about 50 mg/kg body weight of the subject.

14. The use of claim 10, wherein the fibrosis is induced by an antineoplastic agent.

15. The use of claim 10, wherein the fibrosis is induced by radiation.

16. The use of claim 14 wherein the antineoplastic agent is bleomycin.

17. The use of claim 10 wherein the composition is suitable for subcutaneous administration.

18. The use of claim 10 wherein the composition is suitable for intravenous administration.

19. The use of claim 10 wherein the composition is suitable for intratracheal administration.

**Patentansprüche**

1. Verwendung von Humanmangansuperoxiddismutase, eines Humanmangansuperoxiddismutaseanalogen oder einer Humanmangansuperoxiddismutasemutante zur Herstellung einer pharmazeutischen Zubereitung zur Linderung von Symptomen bei einem an Synovialentzündung, Arthritis oder Lungenfibrose leidenden Patienten.

2. Verwendung nach Anspruch 1, wobei der Patient an Synovialentzündung leidet und die Zubereitung sich zur intraartikulären Verabreichung eignet.

3. Verwendung nach Anspruch 2, wobei die Menge an Humanmangansuperoxiddismutase, Humanmangansuperoxiddismutaseanalogem oder Humanmangansuperoxiddismutasemutante in der Zubereitung zwischen etwa 1 und etwa 200 mg/Gelenk liegt.

4. Verwendung nach Anspruch 1, wobei der Patient an Arthritis leidet und die Symptome eine Entzündung beinhalten.

5. Verwendung nach Anspruch 4, wobei sich die Zubereitung zur täglichen Verabreichung eignet.

6. Verwendung nach Anspruch 5, wobei die Menge an Humanmangansuperoxiddismutase, Humanmangansuperoxiddismutaseanalogem oder Humanmangansuperoxiddismutasemutante in der Zubereitung zwischen etwa 1 und etwa 100 mg/kg Körpergewicht des Patienten liegt.

7. Verwendung nach Anspruch 6, wobei die Menge an Humanmangansuperoxiddismutase, Humanmangansuperoxiddismutaseanalogem oder Humanmangansuperoxiddismutasemutante in der Zubereitung zwischen etwa 3 und etwa 50 mg/kg Körpergewicht des Patienten liegt.

8. Verwendung nach Anspruch 4, wobei sich die Zubereitung zur subkutanen Verabreichung eignet.

9. Verwendung nach Anspruch 4, wobei sich die Zubereitung zur intravenösen Verabreichung eignet.

10. Verwendung nach Anspruch 1, wobei der Patient an Lungenfibrose leidet.

11. Verwendung nach Anspruch 10, wobei sich die Zubereitung zur täglichen Verabreichung eignet.

12. Verwendung nach Anspruch 11, wobei die Menge an Humanmangansuperoxiddismutase, Humanmangansuperoxiddismutaseanalogem oder Humanmangansuperoxiddismutasemutante in der Zubereitung zwischen etwa 1 und etwa 100 mg/kg Körpergewicht des Patienten liegt.

13. Verwendung nach Anspruch 12, wobei die Menge an Humanmangansuperoxiddismutase, Humanmangansuperoxiddismutaseanalogem oder Humanmangansuperoxiddismutasemutante in der Zubereitung zwischen etwa 3 und etwa 50 mg/kg Körpergewicht des Patienten liegt.

14. Verwendung nach Anspruch 10, wobei die Fibrose durch ein antineoplastisches Mittel induziert wurde.

15. Verwendung nach Anspruch 10, wobei die Fibrose durch Bestrahlung induziert wurde.

16. Verwendung nach Anspruch 14, wobei das antineoplastische Mittel Bleomycin ist.

17. Verwendung nach Anspruch 10, wobei sich die Zubereitung zur subkutanen Verabreichung eignet.

18. Verwendung nach Anspruch 10, wobei sich die Zubereitung zur intravenösen Verabreichung eignet.

19. Verwendung nach Anspruch 10, wobei sich die Zubereitung zur intratrachealen Verabreichung eignet.

## Revendications

1. Utilisation de la manganèse superoxyde dismutase humaine, d'un analogue de la manganèse superoxyde dismutase humaine ou d'un mutant de la manganèse superoxyde dismutase humaine pour la production d'une composition pharmaceutique pour alléger les symptômes chez un sujet souffrant d'inflammation synoviale, d'arthrite ou de fibrose pulmonaire.

2. Utilisation selon la revendication 1, dans laquelle le sujet souffre d'inflammation synoviale et dans laquelle la composition convient pour l'administration intra-articulaire.

3. Utilisation selon la revendication 2, dans laquelle la quantité de manganèse superoxyde dismutase humaine, d'analogue de la manganèse superoxyde dismutase humaine ou de mutant de la manganèse superoxyde dismutase humaine dans la composition est comprise entre environ 1 et environ 200 mg/articulation.

4. Utilisation selon la revendication 1, dans laquelle le sujet souffle d'arthrite et les symptômes comprennent une inflammation.

5. Utilisation selon la revendication 4, dans laquelle la composition convient pour l'administration journalière.

6. Utilisation selon la revendication 5, dans laquelle la quantité de manganèse superoxyde dismutase humaine, d'analogue de la manganèse superoxyde dismutase humaine ou de mutant de la manganèse superoxyde dismutase humaine dans la composition est comprise entre environ 1 et environ 100 mg/kg de poids corporel du sujet.

**7.** Utilisation selon la revendication 6, dans laquelle la quantité de manganèse superoxyde dismutase humaine, d'analogue de la manganèse superoxyde dismutase humaine ou de mutant de la manganèse superoxyde dismutase humaine dans la composition est comprise entre environ 3 et environ 50 mg/kg de poids corporel du sujet.

**8.** Utilisation selon la revendication 4, dans laquelle la composition convient pour l'administration sous-cutanée.

**9.** Utilisation selon la revendication 4, dans laquelle la composition convient pour l'administration intraveineuse.

**10.** Utilisation selon la revendication 1, dans laquelle le sujet souffre de fibrose pulmonaire.

**11.** Utilisation selon la revendication 10, dans laquelle la composition convient pour l'administration journalière.

**12.** Utilisation selon la revendication 11, dans laquelle la quantité de manganèse superoxyde dismutase humaine, d'analogue de la manganèse superoxyde dismutase humaine ou de mutant de la manganèse superoxyde dismutase humaine dans la composition est comprise entre environ 1 et environ 100 mg/kg de poids corporel du sujet.

**13.** Utilisation selon la revendication 12, dans laquelle la quantité de manganèse superoxyde dismutase humaine, d'analogue de la manganèse superoxyde dismutase humaine ou de mutant de la manganèse superoxyde dismutase humaine dans la composition est comprise entre environ 3 et environ 50 mg/kg de poids corporel du sujet.

**14.** Utilisation selon la revendication 10, dans laquelle la fibrose est induite par un agent antinéoplasique.

**15.** Utilisation selon la revendication 10, dans laquelle la fibrose est induite par un rayonnement.

**16.** Utilisation selon la revendication 14, dans laquelle l'agent antinéoplasique est la bléomycine.

**17.** Utilisation selon la revendication 10, dans laquelle la composition convient pour l'administration sous-cutanée.

**18.** Utilisation selon la revendication 10, dans laquelle la composition convient pour l'administration intraveineuse.

**19.** Utilisation selon la revendication 10, dans laquelle la composition convient pour l'administration intratrachéale.

Figure 1

```
                 10                  20                  30                  40                  50                  60
                  .                   .                   .                   .                   .                   .
GAA TTC GGC GGC GGC ATC AGC GGC TAA GCC AGC ACT AGC AGC ATG TTG AGC CGG GCA GTG
                                                        Met Leu Ser Arg Ala Val

                 70                  80                  90                 100                 110                 120
                  .                   .                   .                   .                   .                   .
TGC GGC ACC AGC AGG CAG CTG GCT CCG GCT TTG GGC TAT CTG GGC TCC AGG CAG AAG CAC
Cys Gly Thr Ser Arg Gln Leu Ala Pro Ala Leu Gly Tyr Leu Gly Ser Arg Gln Lys His

                130                 140                 150                 160                 170                 180
                  .                   .                   .                   .                   .                   .
AGC CTC CCC GAC CTG CCC TAC GAC TAC GGC GCC CTG GAA CCT CAC ATC AAC GCG CAG ATC
Ser Leu Pro Asp Leu Pro Tyr Asp Tyr Gly Ala Leu Glu Pro His Ile Asn Ala Gln Ile

                190                 200                 210                 220                 230                 240
                  .                   .                   .                   .                   .                   .
ATG CAG CTG CAC CAC AGC AAG CAC CAC GCG GCC TAC GTG AAC AAC CTG AAC GTC ACC GAG
Met Gln Leu His His Ser Lys His His Ala Ala Tyr Val Asn Asn Leu Asn Val Thr Glu

                250                 260                 270                 280                 290                 300
                  .                   .                   .                   .                   .                   .
GAG AAG TAC CAG GAG GCG TTG GCC AAG GGA GAT GTT ACA GCC CAG ATA GCT CTT CAG CCT
Glu Lys Tyr Gln Glu Ala Leu Ala Lys Gly Asp Val Thr Ala Gln Ile Ala Leu Gln Pro

                310                 320                 330                 340                 350                 360
                  .                   .                   .                   .                   .                   .
GCA CTG AAG TTC AAT GGT GGT GGT CAT ATC AAT CAT AGC ATT TTC TGG ACA AAC CTC AGC
Ala Leu Lys Phe Asn Gly Gly Gly His Ile Asn His Ser Ile Phe Trp Thr Asn Leu Ser

                370                 380                 390                 400                 410                 420
                  .                   .                   .                   .                   .                   .
CCT AAC GGT GGT GGA GAA CCC AAA GGG GAG TTG CTG GAA GCC ATC AAA CGT GAC TTT GGT
Pro Asn Gly Gly Gly Glu Pro Lys Gly Glu Leu Leu Glu Ala Ile Lys Arg Asp Phe Gly

                430                 440                 450                 460                 470                 480
                  .                   .                   .                   .                   .                   .
TCC TTT GAC AAG TTT AAG GAG AAG CTG ACG GCT GCA TCT GTT GGT GTC CAA GGC TCA GGT
Ser Phe Asp Lys Phe Lys Glu Lys Leu Thr Ala Ala Ser Val Gly Val Gln Gly Ser Gly

                490                 500                 510                 520                 530                 540
                  .                   .                   .                   .                   .                   .
TGG GGT TGG CTT GGT TTC AAT AAG CAA CGG GGA CAC TTA CAA ATT GCT GCT TGT CCA AAT
Trp Gly Trp Leu Gly Phe Asn Lys Gln Arg Gly His Leu Gln Ile Ala Ala Cys Pro Asn

                550                 560                 570                 580                 590                 600
                  .                   .                   .                   .                   .                   .
CAG GAT CCA CTG CAA GGA ACA ACA GGC CTT ATT CCA CTG CTG GGG ATT GAT GTG TGG GAG
Gln Asp Pro Leu Gln Gly Thr Thr Gly Leu Ile Pro Leu Leu Gly Ile Asp Val Trp Glu

                610                 620                 630                 640                 650                 660
                  .                   .                   .                   .                   .                   .
CAC GCT TAC TAC CTT CAG TAT AAA AAT GTC AGG CCT GAT TAT CTA AAA GCT ATT TGG AAT
His Ala Tyr Tyr Leu Gln Tyr Lys Asn Val Arg Pro Asp Tyr Leu Lys Ala Ile Trp Asn

                670                 680                 690                 700                 710                 720
                  .                   .                   .                   .                   .                   .
GTA ATC AAC TGG GAG AAT GTA ACT GAA AGA TAC ATG GCT TGC AAA AAG TAA ACC ACG ATC
Val Ile Asn Trp Glu Asn Val Thr Glu Arg Tyr Met Ala Cys Lys Lys ---

                730                 740                 750                 760                 770                 780
                  .                   .                   .                   .                   .                   .
GTT ATG CTG ATC ATA CCC TAA TGA TCC CAG CAA GAT AAT GTC CTG TCT TCT AAG ATG TGC

                790                 800                 810
                  .                   .                   .
ATC AAG CCT GGG TAC ATA CTG AAA CCC CCA ATT
```

Figure 2

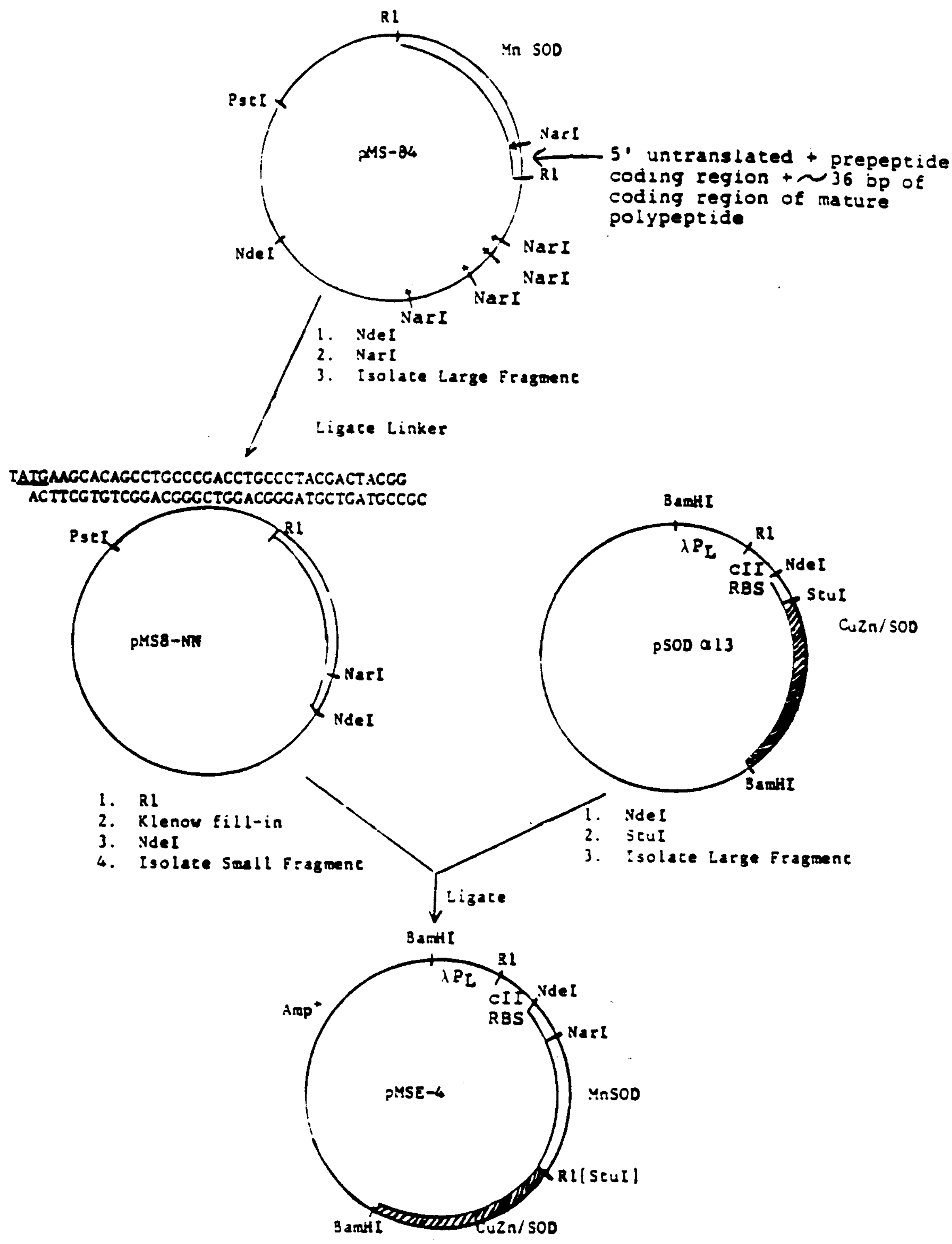
R1
Mn SOD
PstI
NarI
pMS-84
5' untranslated + prepeptide coding region + ~36 bp of coding region of mature polypeptide
R1
NdeI
NarI
NarI
NarI
NarI
1. NdeI
2. NarI
3. Isolate Large Fragment
Ligate Linker
TATGAAGCACAGCCTGCCCGACCTGCCCTACGACTACGG
ACTTCGTGTCGGACGGGCTGGACGGGATGCTGATGCCGC
PstI
R1
pMS8-NN
NarI
NdeI
BamHI
R1
λ$P_L$
cII
NdeI
RBS
StuI
CuZn/SOD
pSOD α13
BamHI
1. R1
2. Klenow fill-in
3. NdeI
4. Isolate Small Fragment
1. NdeI
2. StuI
3. Isolate Large Fragment
Ligate
BamHI
R1
λ$P_L$
cII
NdeI
Amp+
RBS
NarI
pMSE-4
MnSOD
R1(StuI)
BamHI
CuZn/SOD

Figure 3

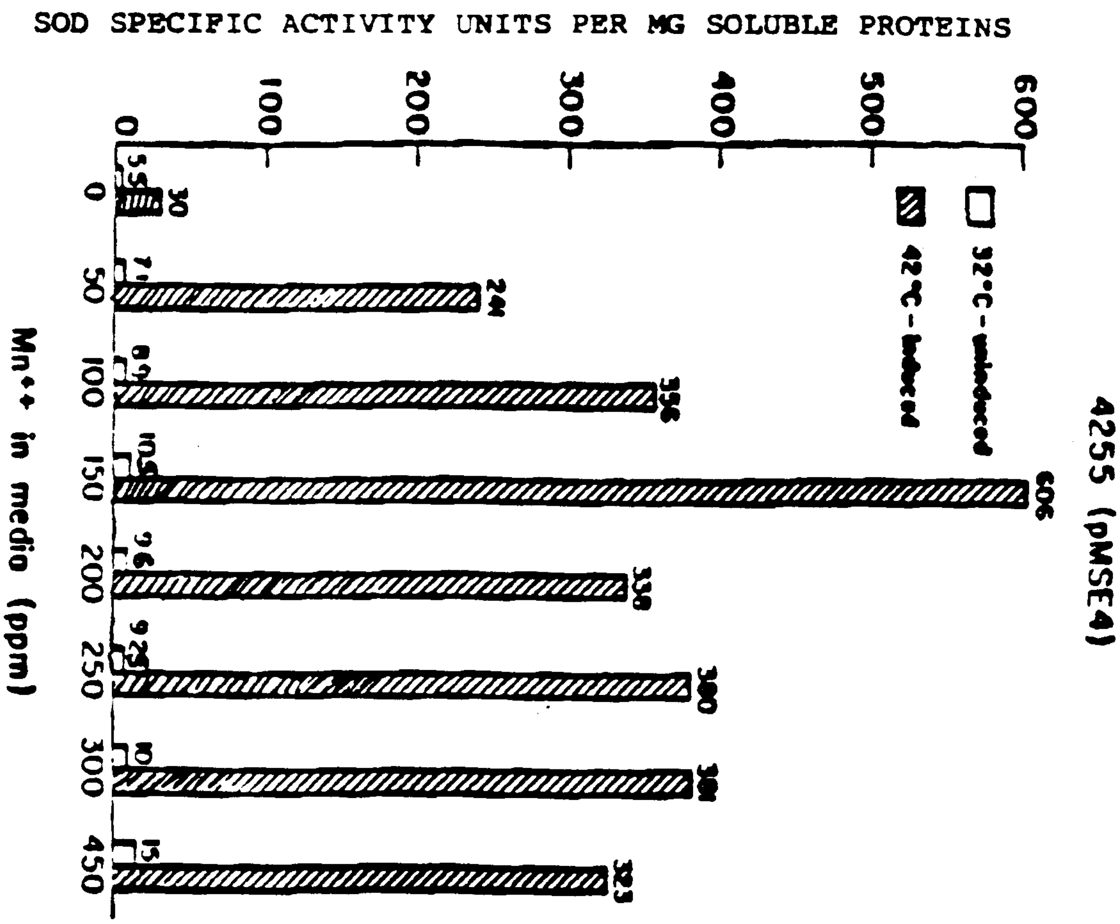
SOD SPECIFIC ACTIVITY UNITS PER MG SOLUBLE PROTEINS
0
100
200
300
400
500
600
4255 (pMSE4)
32°C - uninduced
42°C - induced
Mn++ in medio (ppm)
0
50
100
150
200
250
300
450
30
244
356
606
338
380
384
323
15

# Figure 4

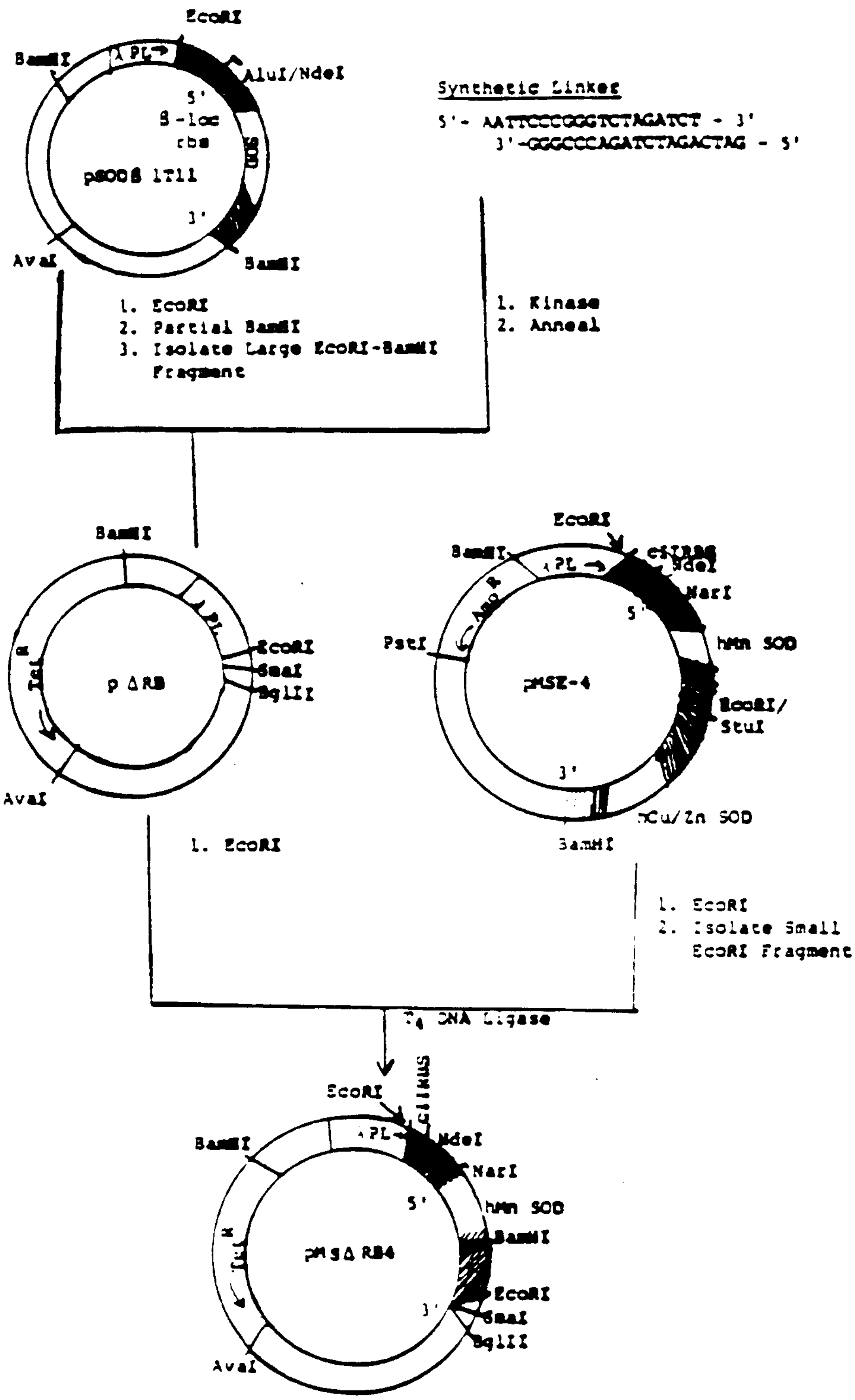
EcoRI
BamHI
λ PL
AluI/NdeI
5'
β-lac
rbs
SOD
pSODβ 1T11
3'
AvaI
BamHI
Synthetic Linker
5'- AATTCCCGGGTCTAGATCT - 3'
3'-GGGCCCAGATCTAGACTAG - 5'
1. EcoRI
2. Partial BamHI
3. Isolate Large EcoRI-BamHI Fragment
1. Kinase
2. Anneal
BamHI
λ PL
EcoRI
SmaI
BglII
Tet
p ΔRB
AvaI
1. EcoRI
EcoRI
BamHI
λ PL
cII RBS
NdeI
NarI
Amp R
PstI
5'
hMn SOD
pMSE-4
EcoRI/
StuI
3'
hCu/Zn SOD
BamHI
1. EcoRI
2. Isolate Small EcoRI Fragment
T4 DNA Ligase
cII RBS
EcoRI
BamHI
λ PL
NdeI
NarI
5'
hMn SOD
BamHI
Tet
pMSΔRB4
3'
EcoRI
SmaI
BglII
AvaI

Figure 5

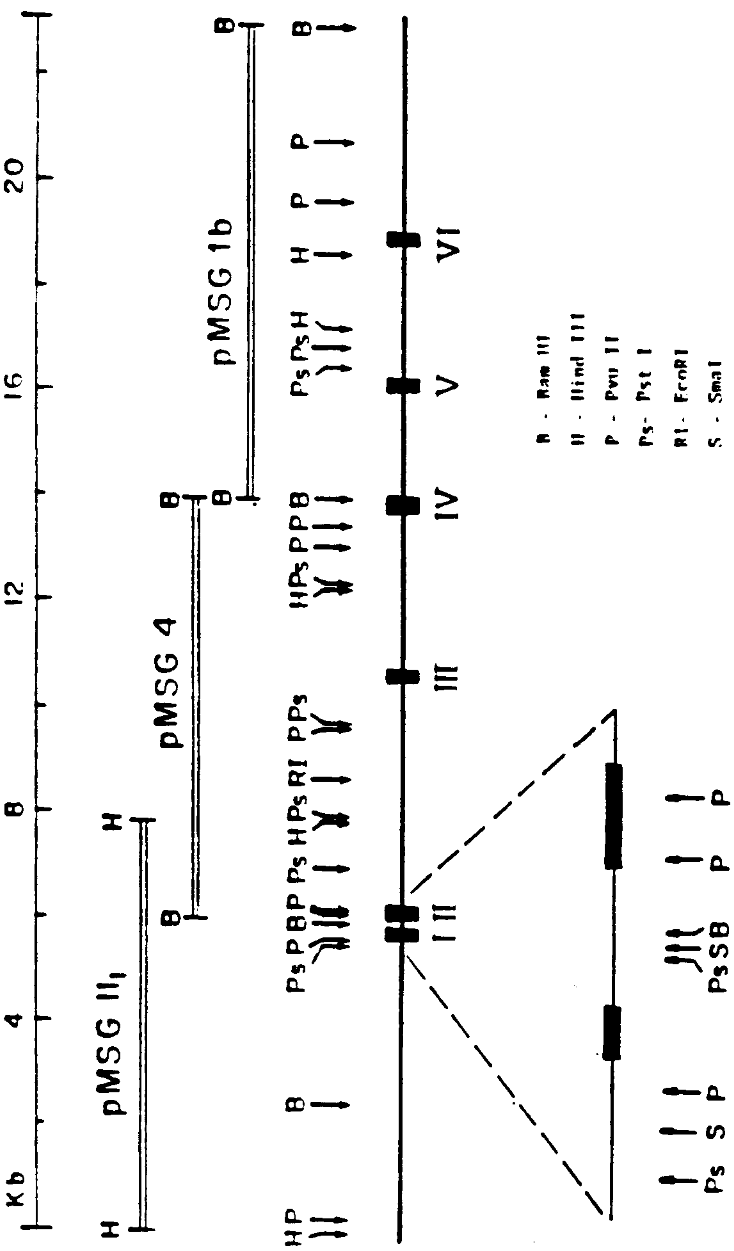


Restriction map and organization of human MnSOD gene.

Figure 6

Nucleotide Sequence of Human MnSOD Gene

1 AACCAAAAAC TCACGGGGCC AGCGCCGGCA GGGCCGCCTA GTGCAGCCAG ATCCCCGCCG

61 GCACCTCAGG GGGCGGACCC GCAGGCAGGG CTTGCGGGCC GTACCAACTG CCACGGGGGC

121 AGGGGCCGCC TCCCTTCGGC CGCGCGCCAC TGCAAGTACG GCAGGACACC AGCGAGGTTG

181 CCGAGGCCGA GGCTAGCCTG CAGCCTCCTT TCTCCCGTGC CCTGGGCGCG GGGTGTACGG

241 CAAGCGCGGG CGGGCGGGAC AGGCACGCAG GGCACCCCCG GGGTTCGGGC GCGGCGGGCG

301 GGGGCGGGGC TCGCGGGGGG AGGGGGCGGG GCGGCGGTCG CCCTTGTCGG CGCAGCTGGG

361 TCGCGGCCCT GCTCCCGGCG CTTTCTTAAG GCCCGCGGGC GGCGCAGGAG CGGTCACTCG

421 TGGCTGTGGT GGCTTCGGCA GCGGCTTCAG CAGATCGGCG GCATCAGCGG TAGCCAGCA
481 CTAGCAGCAT GTTGAGCCGG GCAGTGTGCG GGTGACAAGG AAGGGGACCC GGTCACGCCC
541 CAAGGGGAAG GGGCTCGCGG CGGGCAGGGC TCCGGGCAAT GGCGACATGG CCGCACGGGC
601 CTGGCGGGAC CGCCGACCTG CAGGCGGTTC TCCCGGAGGT GCCCCCGCCG GCGGCCTGGA
661 GCCGGGGATC CGAGGGAGGG GACGCGGGGA CTCGGGGGAC GCCGCGCTGC GTTCCTCGGC
721 AGCCCAGCCT GCGTAGACGG TCCGCGGCGC TGACTGACCG GGCTGTGCTT TCTCGTCTTC
781 AGGCACCAGC AGGCAGCTGG CTCCGGTTTT GGGGTATCTG GGCTCCAGGC AGAAGCACAG
841 CCTCCCCGAC CTGCCCTACG ACTACGGCGC CCTGGAACCT CACATCAACG CGCAGATCAT
901 GCAGCTGCAC CACAGCAAGC ACCACGCGGC CTACGTGAAC AACCTGAACG TCACCGAGGA
961 GAAGTACCAG GAGGCGTTGG CCAAGGGTAG GTTCCAGGCT GAGCGGG... ..........
1021 AAAAAAATGT GGTTTGCACT TTTAACTTTT AAGGAGATGT TACAGCCCAG ATAGCTCTTC
1081 AGCCTGCACT GAAGTTCAAT GGTGGTGGTC ATATCAATCA TAGCATTTTC TGGACAAACC
1141 TCAGCCCTAA CGGTGGTGGA GAACCCAAAG GTTGGATATA TTGTGCACCC TTATCTACAA
1201 CTTCTTGCAC AGTAGGAATC GAT....... .......... .......... ..........
1261 GTAATTTCTT GGGCCCTATG ACAAAAAATA TTTTGAATAC ATGTAATATA ACATTTTACT
1321 GTAATTATTG GAAATCTGTT CATTTGTGGG TGGTTTTGGG ATTTTTTTTT TAATAGGGGA
1381 GTTGCTGGAA GCCATCAAAC GTGACTTTGG TTCCTTTGAC AAGTTTAAGG AGAAGCTGAC
1441 GGCTGCATCT GTTGGTGTCC AAGGCTCAGG TTGGGGTTGG CTTGGTTTCA ATAAGGAACG
1501 GGGACACTTA CAAATTGCTG CTTGTCCAAA TCAGGATCCA CTGCAAGGAA CAACAGGTTA
1561 CATTTAGATA GGGTTGAGTG TTGTTCCAGT TTGGAAAACG AGTCCACTAT TAAAGAACGT
1621 GGACTCCAAC GTCAAAGGGC GAAAAACCGT CTATCAGGGC GATGGCCACT ACGTGAACCA
1681 TCACCCAAAT CAAGTTTTTT GGGGTCGAGG TGCCGTGAAA GCACTAAATC GGAACCTAAA
1741 GGGAGCCCCC GATTTAGAGC TTGACGGGGA AAGCCGGCGG AACTGTGGCG AGAAAGGAAG
1801 GGAAGAAAGC GAAAGGAGCG GGCGCTAGGC GTGGCAATTG TGTAGCGTCA CTGCCG....
1861 CCCCCTTCTT TCTAACAGGC CTTATTCCAC TGCTGGGGAT TGATGTGTGG GAGCACGCTT
1921 ACTACCTTCA GTATAAAAAT GTCAGGCCTG ATTATCTAAA AGCTATTTGG AATGTAATCA
1981 ACTGGGAGAA TGTAACTGAA AGATACATGG CTTGCAAAAA GTAAACCACG ATCGTTATGC
2041 TGAGTATGTT AAGCTCTTTA TGACTGACTA TGTAGTGGTA TAGAGTACTG CAG.......
2101 GTCCATATCT AAAACCACGT ATAAACATTA AATTGTATTT CCTGTTTTAA TTCCAGGGGA
2161 AGTACTGTTT GGCAAAGCTA TTATTAGGTA AATGTTTTAC AAATTACTGT TTCTCAGTTT
2221 CAGTCATACC CTAATGATCC CAGCAAGATA ATGTCCTGTC TTCTAAGATG TGCATCAAGC
2281 CTGGGTACAT ACTGAAACCC TATAAGGTCC CTGGATAATT TTTGTTTGAT TATTGCATTG
2341 AAGAAACATT TATTTTCCAA AATTGTGTGA AGTTTTTGAC TGTTAATAAA AGAATCTGTC
2401 AACCATCAAA GAGGTCTGCA TTAATGCTTG CATGTCTTTT TCATTAAAAA TCCTATAATC
2461 TTCTGTCATT TTCACTGAGT TTCCATGGGA AAGGAATAGT AAACTAATGG GTAGTTGAAA
2521 TATTACTCTT AAGACCAAGA CCTGTGTCTC CAGTCATATC TGTAATAACA TCATCTGATA
2581 ACCTAAAAGC ATAGTATTAG GGATATACGA CAAAACCAAA GTGTTTTTGC TGTTGTCACA
2641 TACCACTCAA TACTTTTACA CCAGTTTGTC CAGTGGGACT CCAGCTG... ..........

Figure 7

Exon-Intron Junctions of Human MnSOD Gene

| | exon | intron | exon |
|---|---|---|---|
| Intron # 1 | TGTGCG | GGTGAG...CTTCAG | GCACCA |
| Intron # 2 | CCAAGG | GTAGGT...TTTAAG | GAGATG |
| Intron # 3 | CCAAAG | GTTGGA...TAATAG | GGGAGT |
| Intron # 4 | CAACAG | GTTACA...TAACAG | GCCTTA |
| Intron # 5 | TGCTGA | GTATGT...TTTCAG | TCATAC |
| | | A TT | |
| CONSENSUS | AG | GT AGT... NCAG | G |
| | | G CC | |

Figure 8

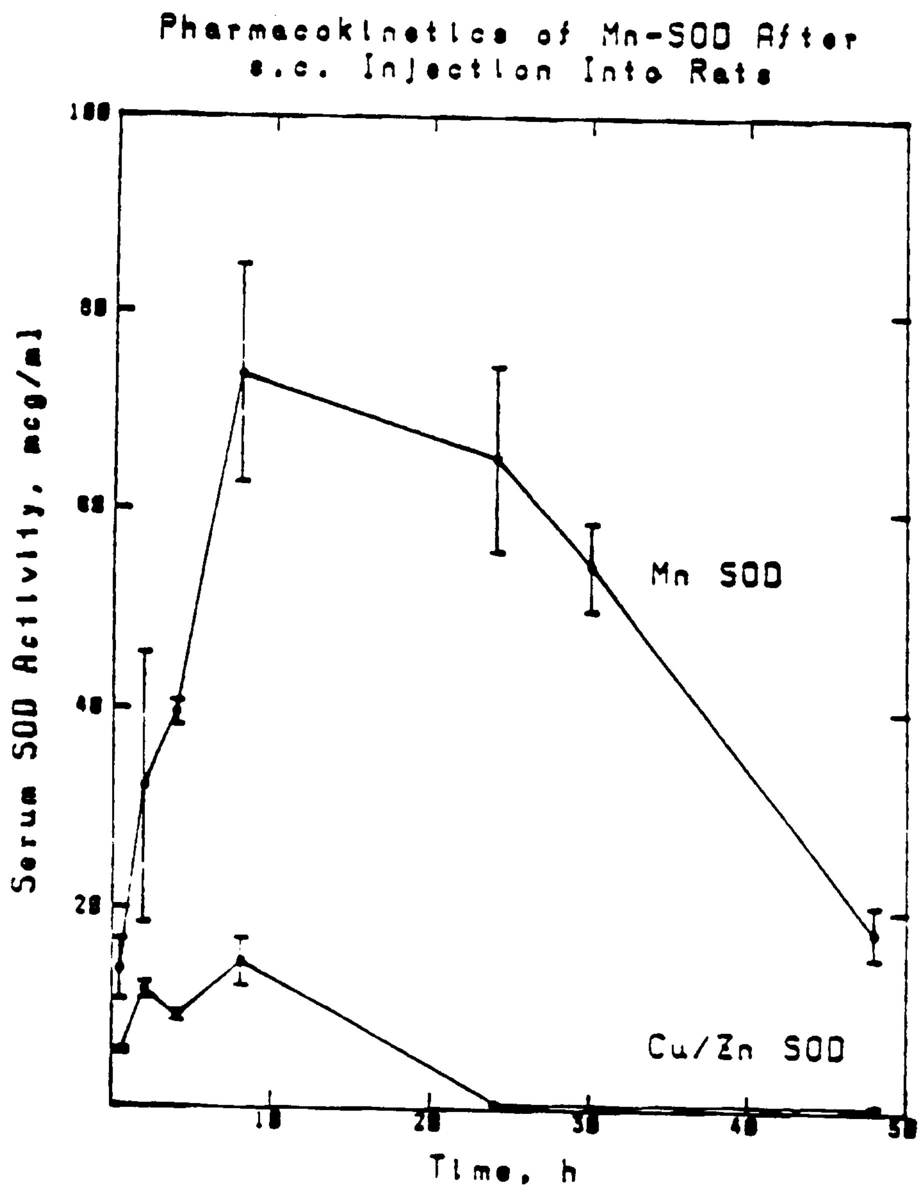
Pharmacokinetics of Mn-SOD After
s.c. Injection Into Rats
Serum SOD Activity, mcg/ml
100
80
60
40
20
10
20
30
40
50
Time, h
Mn SOD
Cu/Zn SOD

Figure 9

EFFECT OF MnSOD ADMINISTERED 24 HOURS BEFORE INSULT AND COMPARISON WITH CU/ZN SOD ANALOG ADMINISTERED AT -2 HOURS AND -24 HOURS

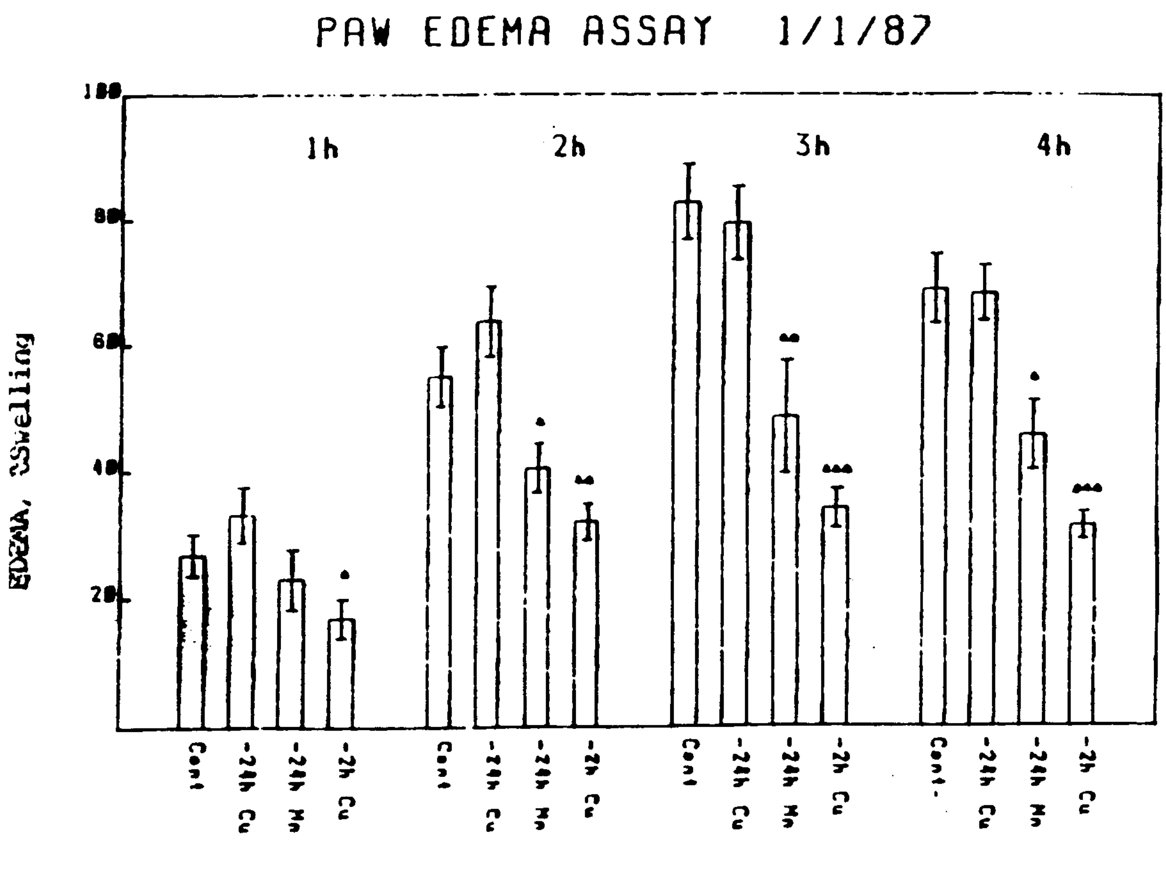

Figure 10

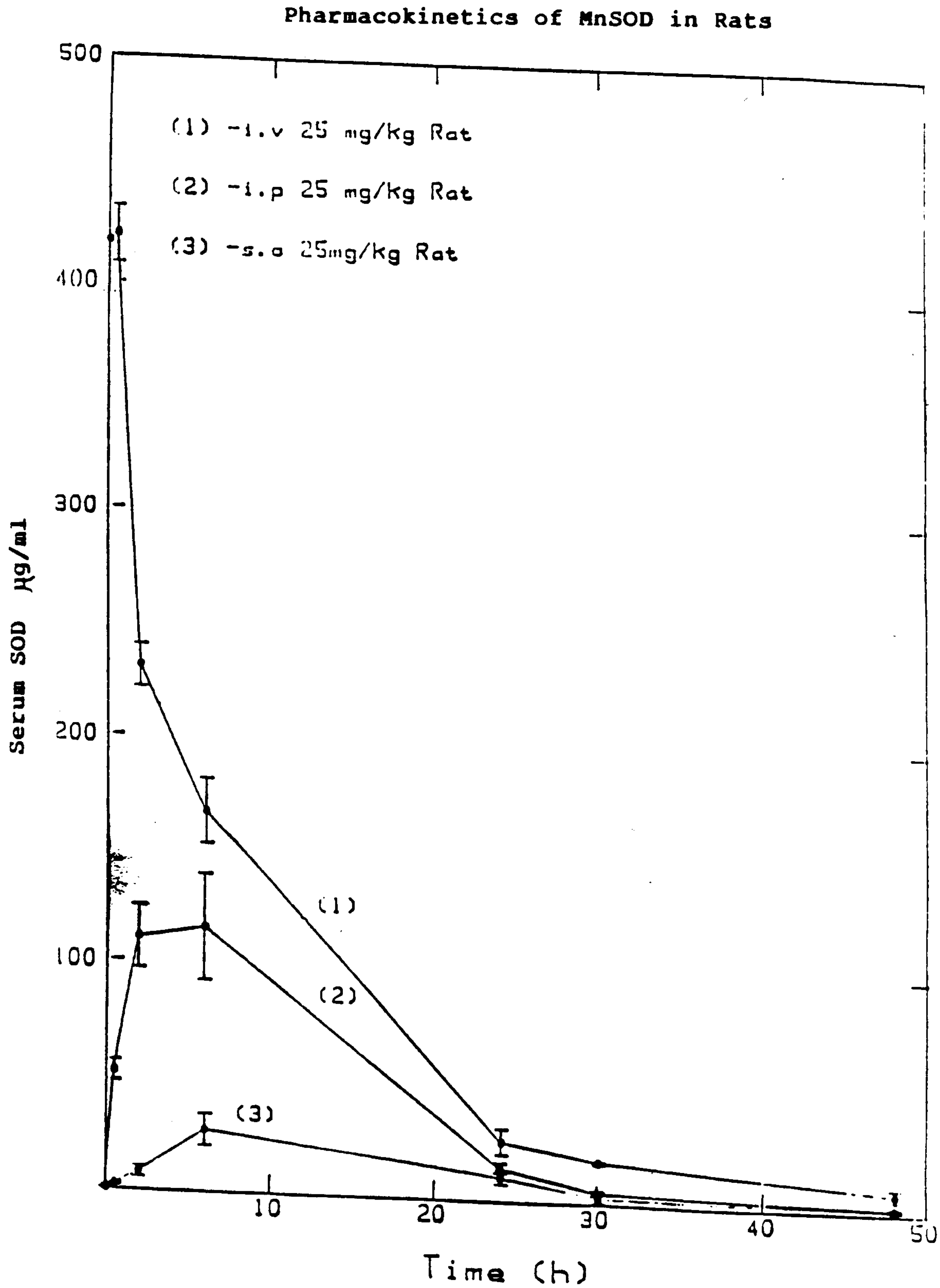
Pharmacokinetics of MnSOD in Rats
(1) -i.v 25 mg/kg Rat
(2) -i.p 25 mg/kg Rat
(3) -s.c 25mg/kg Rat
Serum SOD µg/ml
500
400
300
200
100
(1)
(2)
(3)
10
20
30
40
50
Time (h)

Figure 11

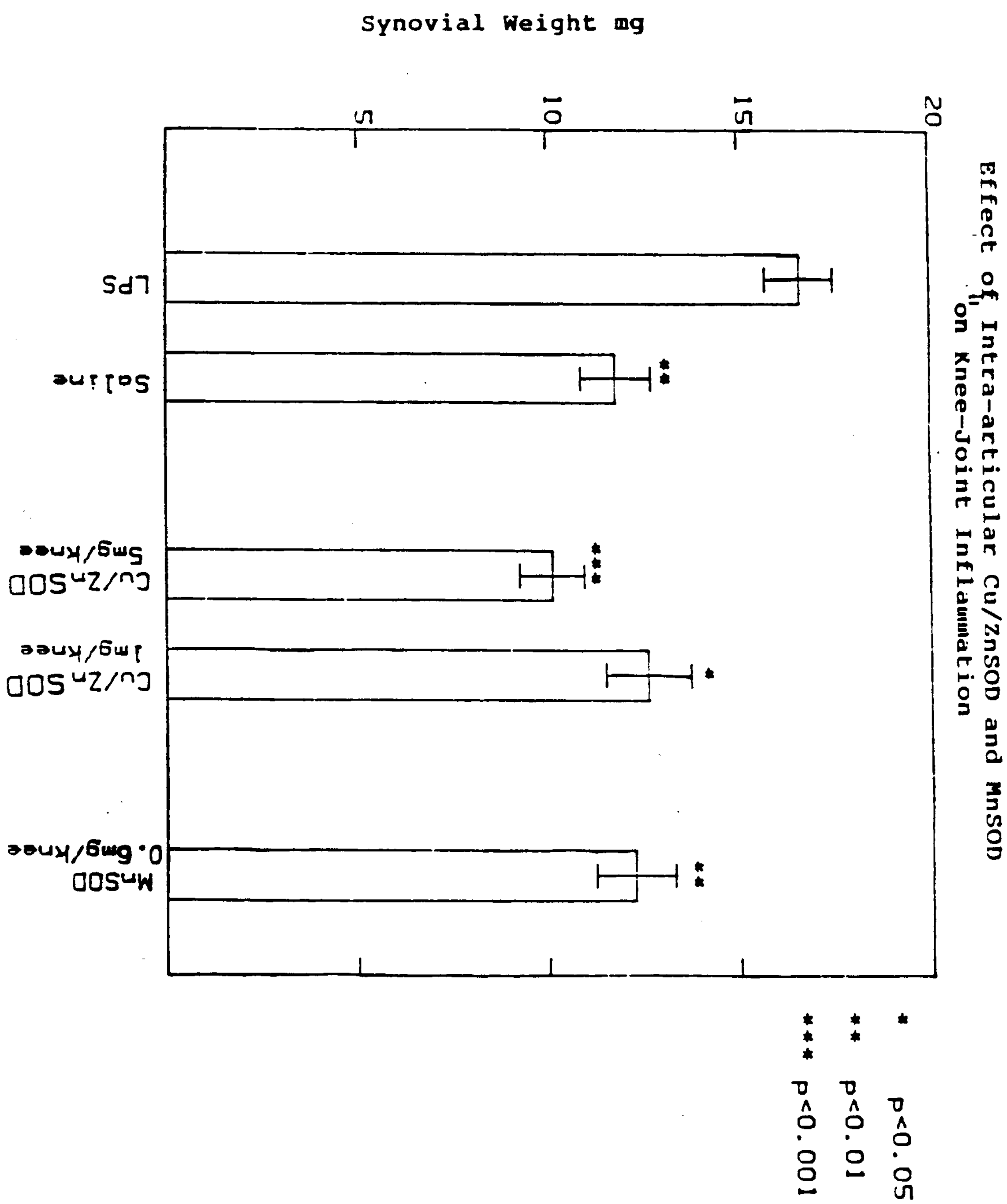
Effect of Intra-articular Cu/ZnSOD and MnSOD on Knee-Joint Inflammation
Synovial Weight mg
5
10
15
20
LPS
Saline
Cu/Zn SOD 5mg/knee
Cu/Zn SOD 1mg/knee
MnSOD 0.6mg/knee
* p<0.05
** p<0.01
*** p<0.001

Figure 12

Effect of CuZnSOD and MnSOD on Adjuvant Arthritis

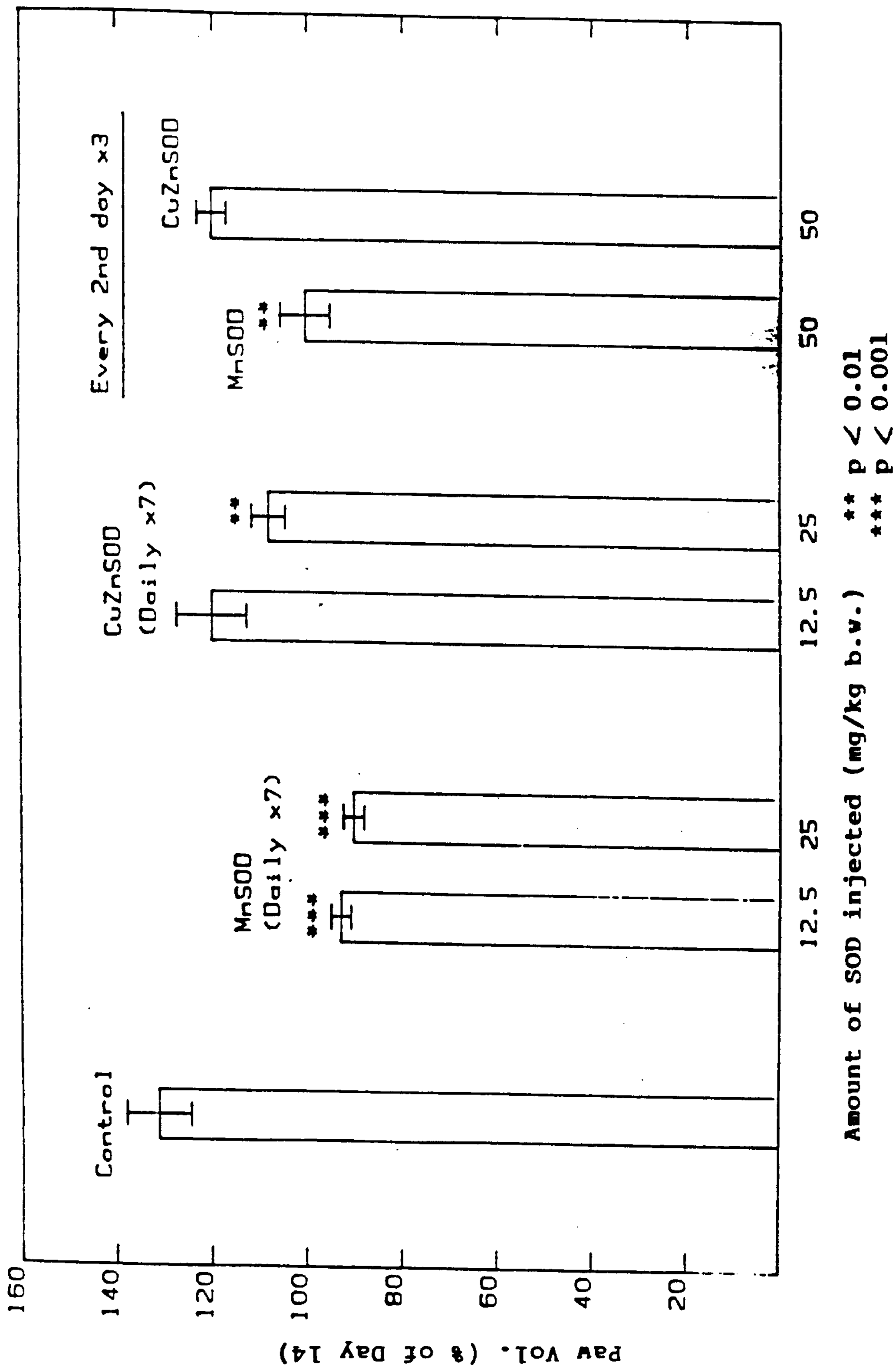

Figure 13

Effect of MnSOD on Bleomycin-induced
Lung Fibrosis

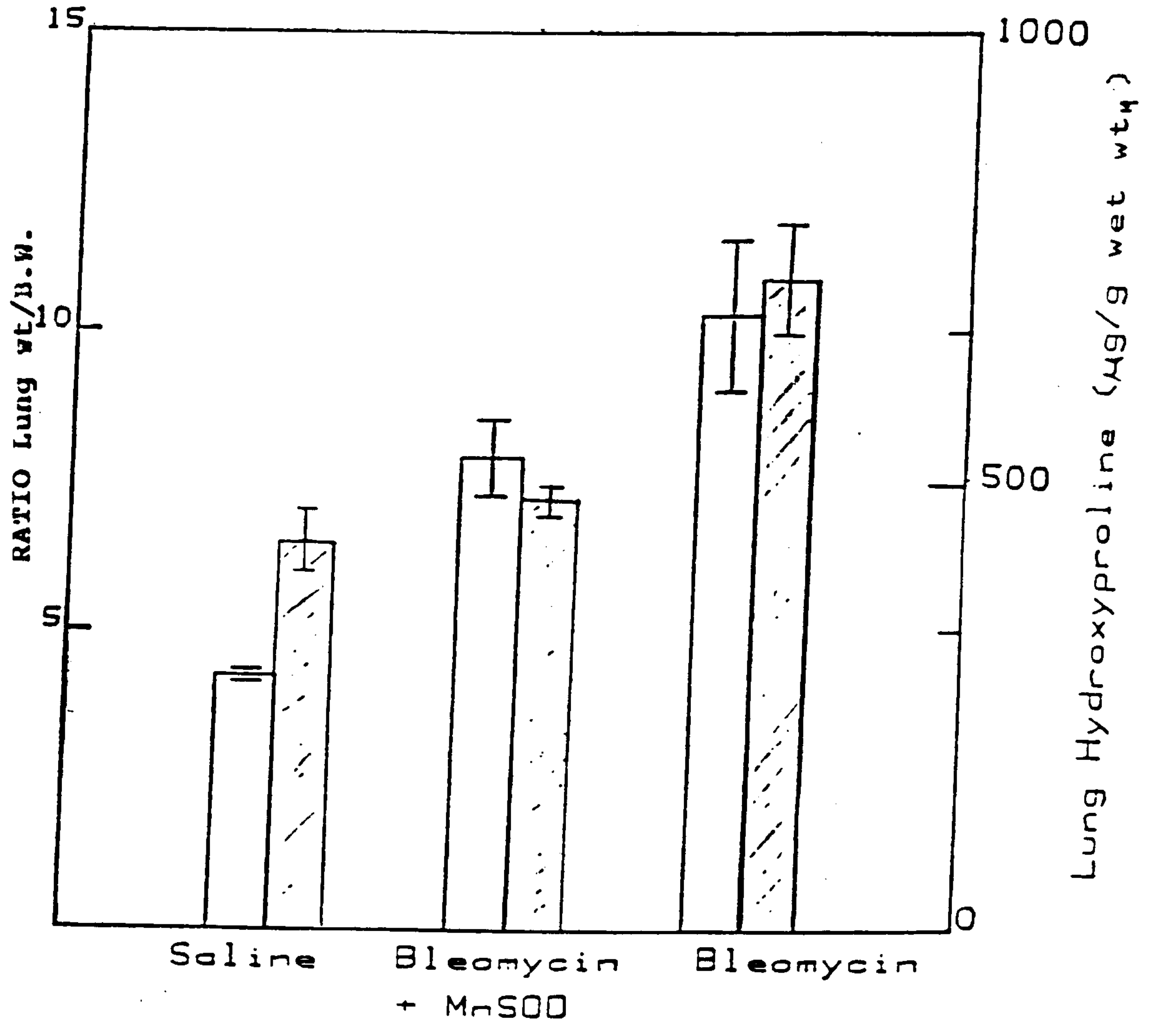

Figure 14

Effect of Daily S.C. Injections
of MnSOD on Lung OH-Proline

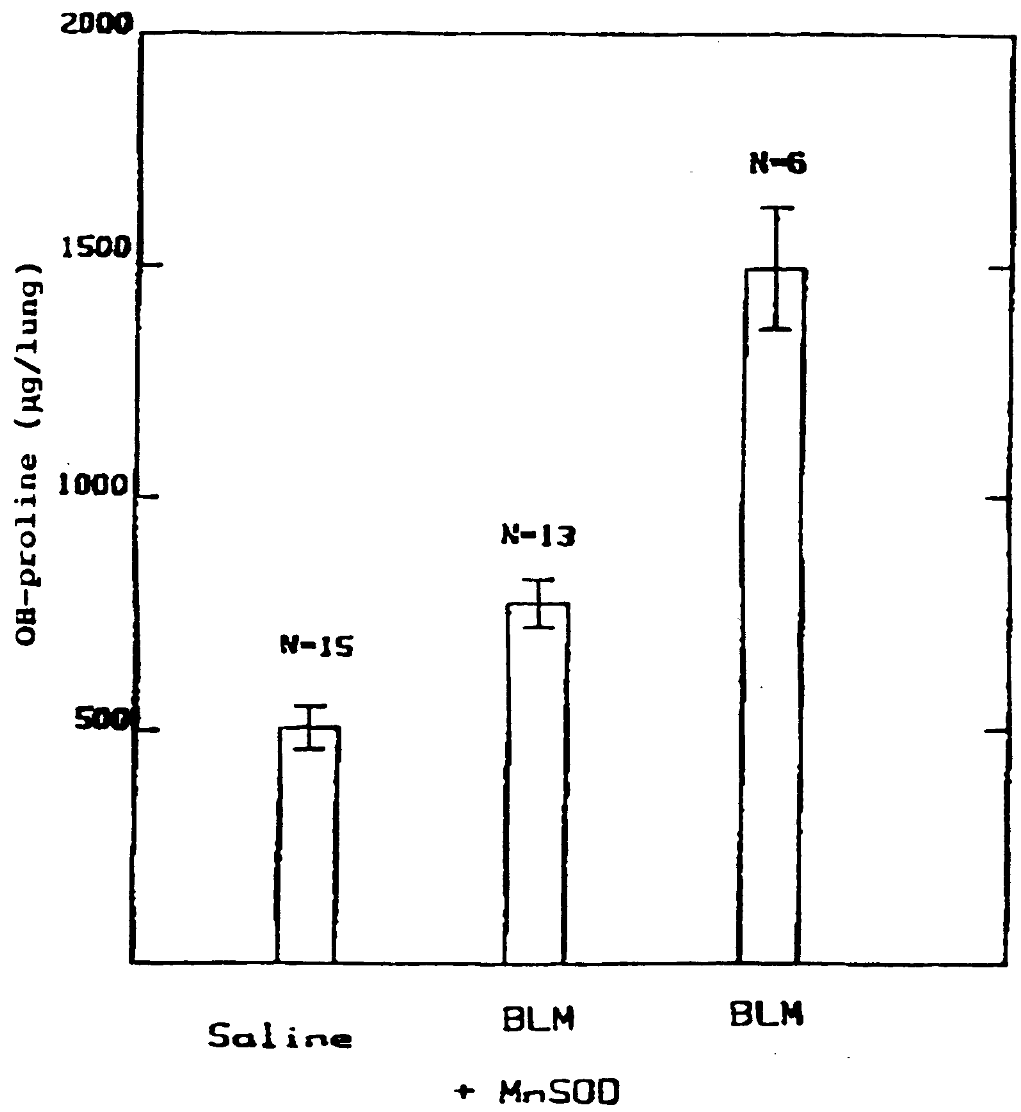

Figure 15

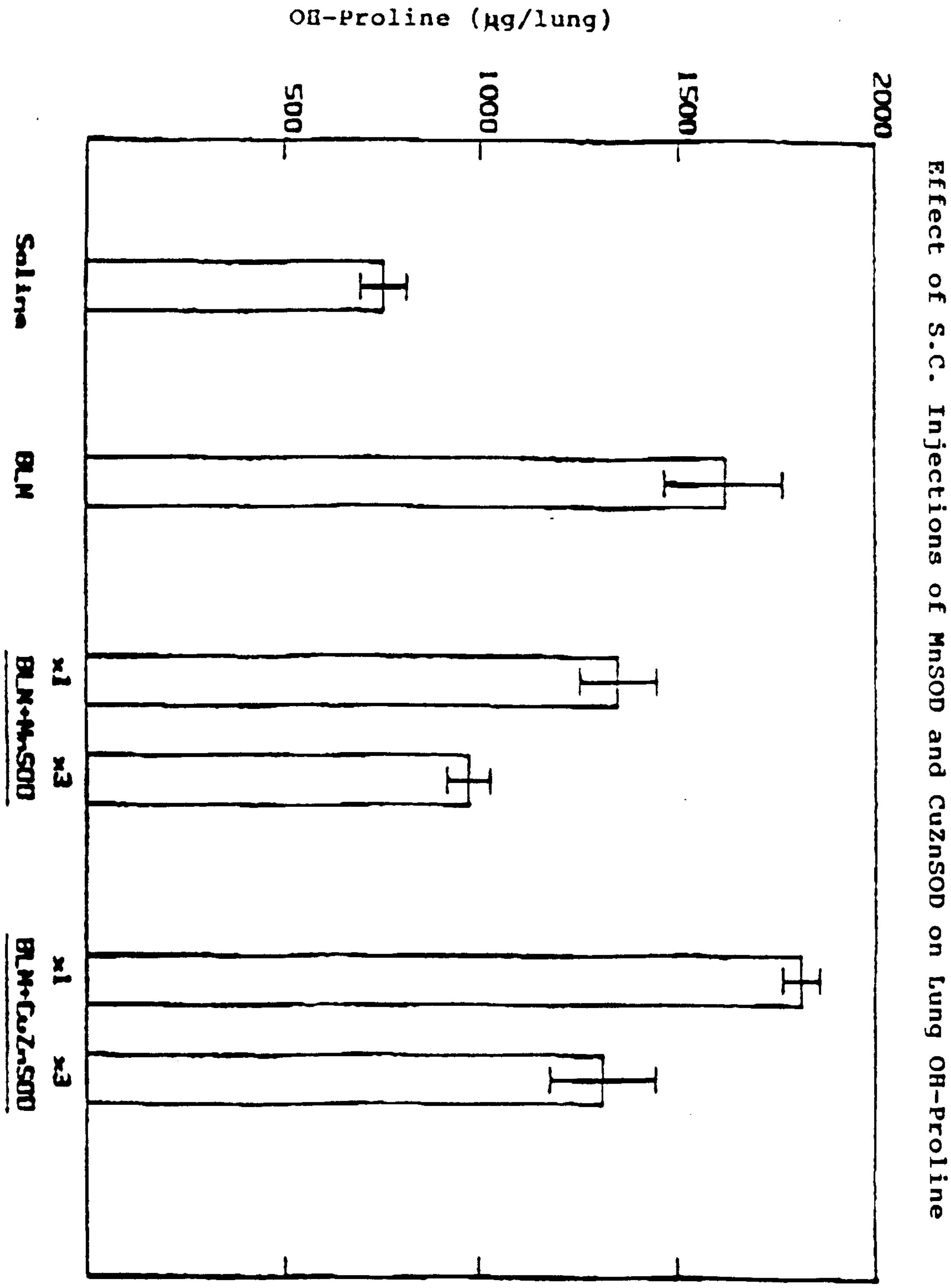
Effect of S.C. Injections of MnSOD and CuZnSOD on Lung OH-Proline
OH-Proline (μg/lung)
500
1000
1500
2000
Saline
BLM
x1
x3
BLM+MnSOD
x1
x3
BLM+CuZnSOD

Figure 16

**Effect of I.P. Injections of MnSOD and CuZnSOD on Lung OH-Proline**

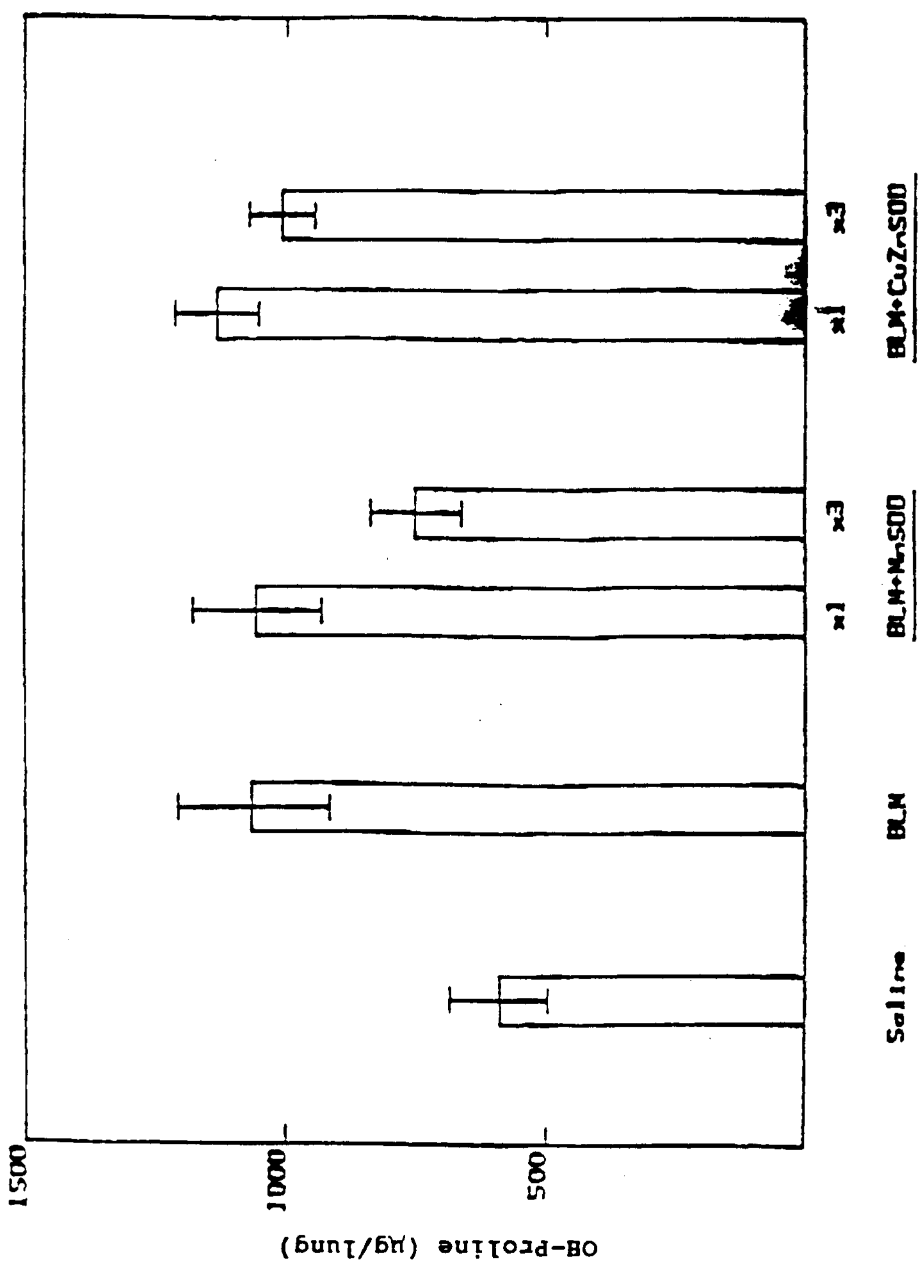


**NOTE:** x1 and x3 represent once a week or three times a week, respectively.